(19) Europäisches Patentamt
European Patent Office
Office européen des brevets

(11) **EP 3 403 101 B1**

(12) **FASCICULE DE BREVET EUROPEEN**

(45) Date de publication et mention de la délivrance du brevet:
**19.10.2022 Bulletin 2022/42**

(21) Numéro de dépôt: **17711698.5**

(22) Date de dépôt: **05.01.2017**

(51) Classification Internationale des Brevets (IPC):
**G01N 35/00** (2006.01)    **G01N 33/569** (2006.01)
**G01N 11/10** (2006.01)

(52) Classification Coopérative des Brevets (CPC):
**G01N 33/56911; G01N 11/10; G01N 35/0098**

(86) Numéro de dépôt international:
**PCT/FR2017/050033**

(87) Numéro de publication internationale:
**WO 2017/121946 (20.07.2017 Gazette 2017/29)**

(54) **PROCÉDÉ DE CLASSIFICATION DE MICROORGANISMES**

VERFAHREN ZUR KLASSIFIZIERUNG VON MIKROORGANISMEN

METHOD FOR CLASSIFYING MICROORGANISMS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.01.2016 FR 1650345**
**31.03.2016 FR 1652784**

(43) Date de publication de la demande:
**21.11.2018 Bulletin 2018/47**

(73) Titulaire: **Biofilm Control**
**63360 Saint-Beauzire (FR)**

(72) Inventeurs:
• **DI DOMENICO, Enea Gino**
**00060 Rome (IT)**
• **ENSOLI, Fabrizio**
**00141 Rome (IT)**
• **TOMA, Luigi**
**00060 Rome (IT)**
• **BERNARDI, Thierry**
**63170 Perignat Les Sarlieve (FR)**
• **PROVOT, Christian**
**63730 Les Martres De Veyre (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
WO-A1-2012/001312    WO-A1-2014/155020
WO-A2-2013/165615    FR-A1- 2 615 621
FR-A1- 2 866 707    FR-A1- 2 898 363
FR-A1- 2 916 761    FR-A1- 2 928 656

• CHAVANT ET AL: "A new device for rapid evaluation of biofilm formation potential by bacteria", JOURNAL OF MICROBIOLOGICAL METHODS, ELSEVIER, AMSTERDAM, NL, vol. 68, no. 3, 16 février 2007 (2007-02-16), pages 605-612, XP005892101, ISSN: 0167-7012, DOI: 10.1016/J.MIMET.2006.11.010
• MARTÍN-ESPADA M C ET AL: "Peracetic acid disinfectant efficacy againstPseudomonas aeruginosabiofilms on polystyrene surfaces and comparison between methods to measure it", LWT- FOOD SCIENCE AND TECHNOLOGY, vol. 56, no. 1, avril 2014 (2014-04), pages 58-61, XP028800477, ISSN: 0023-6438, DOI: 10.1016/J.LWT.2013.11.013
• G Besciak ET AL: "BIOFILM AS A BASIC LIFE FORM OF BACTERIA", , 1 janvier 2012 (2012-01-01), 2011, XP055319775, Extrait de l'Internet: URL:https://www.seed.abe.kth.se/polopoly_fs/1.651085!/JPSU17P13.pdf [extrait le 2016-11-15]

EP 3 403 101 B1

- **JOE J HARRISON ET AL: "Microtiter susceptibility testing of microbes growing on peg lids: a miniaturized biofilm model for high-throughput screening", NATURE PROTOCOLS, vol. 5, no. 7, 10 juin 2010 (2010-06-10), pages 1236-1254, XP055319778, GB ISSN: 1754-2189, DOI: 10.1038/nprot.2010.71**
- **AHYA ABDI-ALI ET AL: "Assessment of Biofilm Formation and Resistance to Imipenem and Ciprofloxacin among Clinical isolates of Acinetobacter baumannii in Tehran", JUNDISHAPUR JOURNAL OF MICROBIOLOGY, vol. 7, no. 1, 1 janvier 2014 (2014-01-01) , XP055319752, ISSN: 2008-3645, DOI: 10.5812/jjm.8606**
- **ALI ZAREI MAHMOUDABADI ET AL: "Biofilm Formation and Susceptibility to Amphotericin B and Fluconazole inCandida albicans", JUNDISHAPUR JOURNAL OF MICROBIOLOGY, vol. 7, no. 7, 1 juillet 2014 (2014-07-01) , XP055319761, ISSN: 2008-3645, DOI: 10.5812/jjm.17105**
- **S. Brook Peterson ET AL: "Different Methods for Culturing Biofilms In Vitro" In: "Biofilm Infections", 5 octobre 2010 (2010-10-05), Springer New York, New York, NY, XP055319766, ISBN: 978-1-4419-6084-9 pages 251-266, DOI: 10.1007/978-1-4419-6084-9_15, le document en entier**
- **MAJED MASADEH ET AL: "In vitro determination of the antibiotic susceptibility of biofilm-forming Pseudomonas aeruginosa and Staphylococcus aureus: possible role of proteolytic activity and membrane lipopolysaccharide", INFECTION AND DRUG RESISTANCE, 1 mars 2013 (2013-03-01), page 27, XP055319769, DOI: 10.2147/IDR.S41501**

**Description**

**Domaine technique**

La présente invention se rapporte à procédé de détermination de la capacité de production de biofilm par un microorganisme

La présente invention se rapporte également à un procédé de classification de microorganisme en fonction de leur capacité de production de biofilm.

La présente invention trouve une application notamment dans les domaines analytiques, de la recherche biologique, enzymologique, dans le domaine pharmaceutique, dans le domaine du diagnostic et/ou dans le domaine médical. La présente invention trouve également une application dans les domaines clinique, environnemental ou agro -alimentaire.

Dans la description ci-dessous, les références (auteur, année) renvoient à la liste des références présentée à la fin du texte.

**Etat de la technique**

La formation d'un biofilm est une propriété clé de bactéries, elle permet la survie à long terme à la fois dans les écosystèmes naturels et des animaux hôtes (Høiby, 2010b). En fait, les bactéries qui se développent dans une matrice biofilm sont intrinsèquement plus résistantes aux agents de l'environnement ainsi qu'aux antimicrobiens par rapport aux cellules planctoniques en croissance. Les biofilms bactériens représentent un sérieux problème dans l'industrie et la médecine, y compris la chirurgie et la médecine dentaire, où ils causent souvent des infections chroniques et récurrentes (Lindsay, 2006). Les bactéries à Gram positif et à Gram négatif sont toutes les deux capables de former un biofilm (Donlan, 2001). Il a été estimé que jusqu'à 80% des maladies infectieuses humaines qui ont un impact significatif sur la morbidité et de la santé coûts pour les patients (Romling, 2012; NIH Parent Grant Announcement, 2002) sont liés à des biofilms. La formation de biofilms sur les dispositifs médicaux implantables provoque des infections persistantes, qui représentent plus de 60% des infections nosocomiales rapportées (Darouiche, 2004). Dans ce cadre, les infections liées aux cathéters représentent l'événement indésirable le plus grave et coûteux causés par un biofilm produit par des bactéries, aboutissant fréquemment à l'échec du traitement et exigeant le retrait du dispositif médical (Costerton et al, 2003). Des exemples typiques de maladies associées au biofilm comprennent les infections «difficiles» telles que les infections osseuses et articulaires, causées par Staphylococcus aureus qui est une bactérie fortement productrice de biofilm (Kurtz, et al., 2012, Jacqueline, 2014, Gbejuade, 2015), les endocardites infectieuses causées principalement par des staphylocoques ou les streptocoques, qui sont associés à des taux de mortalité élevés (Furuya, 2003) et les infections affectant les patients atteints de mucoviscidose, où P. aeruginosa provoque des infections pulmonaires chroniques et une défaillance respiratoire (Singh, 2000; Høiby, 2010a).

Le traitement antibiotique, soit empirique ou sur la base de tests de sensibilité aux médicaments, est souvent incapable de contrôler des infections difficiles liées au biofilm comme dans le cas d'infection sur dispositifs médicaux (Høiby, 2010a). En effet, les profils de résistance aux antibiotiques traditionnels sont réalisés sur des micro-organismes à croissance planctonique. Ainsi, la sensibilité aux antibiotiques in vitro ne prend pas en compte la production de biofilm et pourrait ne pas être représentative de la résistance aux médicaments des bactéries in vivo (Costerton et al., 1999). Par conséquent, de nouveaux tests de laboratoire capables d'évaluer la production de biofilm et la sensibilité de la formation du biofilm aux antibiotiques des microorganismes sont nécessaires et souhaitables en plus à des tests de microbiologie de routine afin de cibler efficacement les agents infectieux difficiles.

Le document Chavant et al, Elsevier Amsterdam vol 68, no 3, 16 février 2007, pages 605-612 décrit un procédé permettant de déterminer si une bactérie forme ou non un biofilm, en d'autres termes l'enseignement de ce document est un procédé de détection de biofilm ou un procédé permettant de déterminer si un microorganisme produit un biofilm. Ce document décrit qu'une variation de BFI inférieure à 2 différence entre la condition test et la condition contrôle signifie pas de formation de biofilm et une variation de BFI supérieure à 20 correspond à la présence d'un biofilm (page 607, colonne de gauche, 1er paragraphe).

Le document FR 2916 761 décrit des tests antibiogrammes. Plus particulièrement, ce document décrit un procédé de détermination de la sensibilité d'un microorganisme à un antibiotique susceptible d'empêcher le développement d'un biofilm, le procédé comprenant l'introduction dans un milieu de culture ensemencé avec un microorganisme au moins une particule magnétique ou magnétisable, l'ajout dans ledit milieu d'un antibiotique puis le maintien du milieu de culture dans des conditions permettant le développement du microorganisme, la sensibilité du microorganisme audit antibiotique est notamment déterminé par application d'un champ magnétique destiné à mettre en mouvement ladite particule sur une surface immergée dans ledit milieu de culture ou à la surface dudit milieu de culture en l'absence de biofilm.

Le document FR 2 866 707 décrit un procédé permettant de mesurer la viscosité d'un milieu de culture de microorganismes comprenant les étapes consistant successivement à :a) immerger au moins une particule chargée électriquement ou magnétique, magnétisable ou revêtue d'au moins une couche magnétique ou magnétisable dans la culture ,b) soumettre la culture à un champ électrique ou magnétique ou électromagnétique de façon à mettre ladite particule en mouvement, c) détecter le degré de liberté de mouvement de la particule dans la culture.

Le document Martin-espada et al. Food science and technology, vol 56, no 1, pages 58-61 à pour objet la détermination de l'efficacité d'un produit antibiofilm. En particulier, ce document décrit notamment un procédé comprenant l'incubation de bactéries (100CFU.ml$^{-1}$) afin de former un biofilm, l'ajout du produit antibiofilm (PERAsafe (marque de commerce)) et incubation du mélange, les bactéries survivantes sont récupérées après rinçage et sonication et des échantillons sont préparés.

Le document G Besciak, janvier 2012, décrit l'observation de biofilms pour une caractérisation de sa structure. En particulier, ce document décrit notamment deux incubations de bactéries afin de former un biofilm et une quantification des bactéries ayant formé un biofilm, après grattage, remise en suspension et étalement sur boite de pétri.

Le document Joe J Harrison et al. Nature protocols, vol.5, no 7, juin 2010, page 1236-1254 décrit un support particulier pour étudier les biofilms, et en particulier une susceptibilité des biofilms à des antimicrobiens (page 1237, colonne de gauche, 2ème paragraphe). Ce document décrit également la détermination du nombre de cellules viables (VCC). Ce document décrit également la détermination de la taille de l'inoculum utilisant des standards Mc Farland.

Il existe donc un réel besoin de trouver un nouveau procédé permettant d'évaluer la production de biofilm par un microorganisme ainsi que sa sensibilité éventuelle dans la formation du biofilm ou du biofilm formé aux antibiotiques.

En outre, il existe un réel besoin de trouver un procédé facile à mettre en œuvre et permettant de caractériser rapidement la capacité productrice de biofilm du microorganisme.

Une technique de diagnostic idéale doit être capable de fournir une identification rapide du micro-organisme (s), pour aider à orienter une thérapie antimicrobienne appropriée.

Une variété de méthodes quantitatives, directes ou indirectes, ont été développées pour évaluer la capacité des souches bactériennes à adhérer aux surfaces (Peeters, 2008), basées soit sur la colorimétrie (Stepanovic, 2000; Joseph, 2004) ou sur des techniques microscopiques (Sangetha, 2009;. Benoit, 2010; Müsken, 2010). À l'heure actuelle, la coloration au cristal violet (CV) est la méthode la plus largement utilisée pour la quantification in vitro de biofilm, en raison de sa relative simplicité et sa sensibilité (Christensen, 1985; Stepanovic, 2000). Cette méthode présente cependant des limitations importantes. En fait, il faut habituellement au moins 24/48 heures d'incubation et des mesures répétées de traitement, qui conduisent à des résultats avec un grand écart-type, et rendent la méthode ni facilement réalisable pour la normalisation, ni adaptable à du criblage à grande échelle dans le cadre clinique. En outre, cette méthode donne des résultats de CMI pour éradiquer un biofilm après 24/48 heures impliquant de très fortes concentrations d'antibiotiques sans pertinence clinique. En d'autres termes les concentrations minimales inhibitrices (CMI) sont tellement importantes qu'elles ne peuvent être utilisées en clinique. Récemment, une nouvelle technologie, à savoir le Biofilm Ring Test® (BFRT), a été proposée pour l'évaluation de biofilm bactérien. Le principe est basé sur l'immobilisation de billes magnétiques par la matrice de film biologique en croissance in vitro (Chavant, 2007). Ce procédé ne nécessite pas de manipulation extensive (par exemple ne nécessite pas d'étapes de lavage ni de coloration), ce qui assure ainsi une reproductibilité des résultats et réduit l'écart-type, un critère positif pour la normalisation des procédures. Ainsi, le BFRT représente un outil prometteur pour la détection d'un biofilm dans le cadre clinique. Toutefois, ce procédé permet uniquement de déterminer la présence ou non d'un biofilm. En outre, ce procédé ne permet pas, notamment dans une seule détermination, de fournir des informations directes sur la "dynamique" et la "force" d'un micro-organisme à produire du biofilm, en particulier par rapport à une norme de référence d'isolats microbiens.

En effet, la procédure initiale peut nécessiter des mesures répétées, effectuées à différents temps, afin d'estimer la formation du biofilm microbien, constituant une autre limitation importante pour une utilisation de routine dans des tests de laboratoire.

Il existe donc un réel besoin de trouver un moyen/procédé permettant de déterminer la dynamique / capacité de production de biofilm d'un microorganisme.

Il existe également des procédés, notamment pour application en diagnostic permettant de déterminer la capacité d'antibiotique à inhiber la formation de biofilm. Il s'agit notamment du procédé antibiofilmogram (marque déposée) dans un format où par exemple 11 antibiotiques, à 8 doses différentes sont prêts à l'emploi dans des microplaques (Olivares, 2015). Toutefois, ce procédé connu et couramment utilisé en diagnostique ne permet pas de caractériser ni de déterminer les capacités productrices de biofilm de microorganismes.

Il existe donc un réel besoin de trouver un procéder palliant ces défauts, inconvénients et obstacles de l'art antérieur, en particulier d'un procédé permettant notamment de déterminer la production de biofilm et de déterminer les capacités de production de biofilm d'un microorganisme permettant par exemple d'adapter les procédés de traitement en fonction des caractéristiques du microorganisme.

En outre, il existe un réel besoin de trouver un procédé simple, peu couteux et applicable en clinique permettant d'évaluer, notamment la capacité éventuelle de production de biofilms par un microorganisme.

Il existe également un réel besoin de trouver un moyen/procédé permettant de déterminer dans un temps court la capacité de production de biofilm par un microorganisme.

**Description de l'invention**

La présente invention permet de résoudre ces problèmes et inconvénients de l'état de la technique en fournissant un procédé de détermination de la capacité de production de biofilm par un microorganisme comprenant les étapes suivantes:

a) Introduire dans des contenants de culture comprenant un milieu de culture liquide approprié au développement dudit microorganisme au moins deux particules, lesdites particules reposant sur une surface immergée dans le milieu de culture,

b) Introduire et ensemencer indépendamment le milieu de culture des contenants obtenus à l'étape a) avec ledit microorganisme à une concentration comprise préférentiellement de $1 \times 10^{-1}$ à $1 \times 10^{-6}$ McFarland (McF), chaque milieu comprenant indépendamment une concentration différente de microorganismes,

c) maintenir les milieux de culture ensemencés dans des conditions permettant un développement dudit microorganisme,

d) appliquer un champ capable de mettre en mouvement lesdites au moins deux particules reposant sur une surface immergée dans le milieu de culture, afin qu'elles s'agrègent préférentiellement sous forme d'un spot

e) détermination de la capacité de production de biofilm par le microorganisme par observation et/ou mesure de l'agrégation desdites particules comme suit :

- l'absence d'agrégation desdites particules apparaissant à une concentration comprise de $1 \times 10^{-6}$ à $1 \times 10^{-4}$ McF correspondant à un microorganisme fort producteur de biofilm,
- l'absence d'agrégation desdites particules apparaissant à partir d'une concentration supérieure à $1 \times 10^{-4}$ et inférieure à $1 \times 10^{-2}$ McF correspondant à un microorganisme producteur intermédiaire de biofilm,
- l'absence d'agrégation apparaissant à partir d'une concentration supérieure ou égale à $1 \times 10^{-2}$ McF correspondant à un microorganisme faible producteur de biofilm,
- une agrégation desdites particules quelle que soit la concentration correspondant à un microorganisme non producteur de biofilm

Les inventeurs ont démontré de manière surprenante que le procédé selon l'invention permet avantageusement et dans un temps très court, par exemple inférieur à 6 heures, de déterminer la capacité de production de biofilm d'un microorganisme et/ou de classifier les microorganismes en fonction de leur capacité de production de biofilm.

En particulier, les inventeurs ont démontré, conformément aux connaissances générales, qu'il y a au moins quatre catégories de microorganismes, à savoir notamment des microorganismes ne produisant pas de biofilm, des microorganismes produisant peu de biofilm (faibles producteurs), des microorganismes moyen producteur de biofilm (producteurs intermédiaires), et des microorganismes fort producteurs de biofilm.

Les inventeurs ont également démontré de manière surprenante que le procédé selon l'invention repose sur la mesure de la formation de biofilm à un stade précoce. En particulier, ils ont démontré que, dans une période de temps donnée, par exemple 5 heures, plus les concentrations initiales de microorganismes, par exemple de cellules bactériennes, qui peuvent inhiber le mouvement/agrégation des particules sont faibles, plus forte est leur capacité à produire un biofilm. Inversement, si des concentrations élevées de microorganismes, par exemple de bactéries, ne sont pas en mesure d'empêcher le mouvement/agrégation des microparticules, ils sont considérés comme à faible risque/non producteurs de biofilm. En outre, les inventeurs ont évalué et démontré la caractérisation/capacité de formation de biofilm de deux isolats cliniques à Gram positif et à Gram négatif et l'ont comparée à celle des souches de laboratoire avec des phénotypes de biofilms connus ainsi que pour les témoins négatifs, utilisé comme référence interne, pour assurer l'exactitude et la reproductibilité des résultats. La reproductibilité inter-essai a été déterminée par comparaison avec le test de coloration au Cristal Violet (CV).Les inventeurs ont démontré que les résultats obtenus concernant la caractérisation des microorganismes sont cohérents avec ceux obtenus par les techniques usuelles telle que la coloration Cristal Violet dans un temps bien plus court, par exemple 5 à 10 fois plus court.

En outre, les inventeurs ont démontré de manière surprenante que le procédé de l'invention permet d'obtenir un résultat fiable et reproductible avec une spécificité similaire voir supérieure à celle des procédés usuels.

De plus le procédé selon l'invention permet avantageusement de s'affranchir de résultats non spécifiques liés notamment à la production de mucus par les microorganismes contrairement aux procédés usuels, notamment basés sur une révélation avec des colorants tels que le Cristal Violet, qui est connu pour colorer le mucus et les bactéries, surestimant ainsi la présence de biofilm et/ou à l'origine de résultats faux positifs. En particulier, les procédés basés sur une révélation avec des colorants tels que le Cristal Violet ne quantifient donc pas précisément la biomasse bactérienne.

La présente invention a également pour objet un procédé de classification de microorganismes comprenant les étapes suivantes:

a) Introduire dans des contenants de culture comprenant un milieu de culture liquide approprié au développement dudit microorganisme au moins deux particules, lesdites particules reposant sur une surface immergées dans le milieu de culture,

b) Introduire et ensemencer indépendamment le milieu de culture des contenants obtenus à l'étape a) avec ledit microorganisme à une concentration comprise de $1 \times 10^{-1}$ à $1 \times 10^{-6}$ McF, chaque milieu comprenant indépendamment une concentration différente de microorganismes,

c) maintenir les milieux de culture ensemencés dans des conditions permettant un développement dudit microorganisme,

d) appliquer un champ capable de mettre en mouvement lesdites au moins deux particules reposant sur une surface immergée dans le milieu de culture, afin qu'elles s'agrègent préférentiellement sous forme d'un spot

e) classification du microorganisme par observation et/ou mesure de l'agrégation desdites particules comme suit:

- catégorie I : absence d'agrégation des particules apparaissant à une concentration comprise de $1 \times 10^{-6}$ à $1 \times 10^{-4}$ McF, correspondant à un microorganisme fort producteur de biofilm
- catégorie II : absence d'agrégation des particules apparaissant à partir d'une concentration supérieure à $1 \times 10^{-4}$ et inférieure à $1 \times 10^{-2}$ McF correspondant à un microorganisme producteur intermédiaire de biofilm,
- catégorie III: absence d'agrégation apparaissant à partir d'une concentration supérieure ou égale à $1 \times 10^{-2}$ McF correspondant à un microorganisme faible producteur de biofilm,
- catégorie IV : agrégation des particules quelle que soit la concentration correspondant à un microorganisme non producteur de biofilm.

Dans la présente, par unité McFarland (McF) on entend l'unité internationale connue de l'homme du métier, par exemple tel que décrit notamment dans le document « National Commitee for Clinical Laboratory Standards », 2001 et 2003.

Dans la présente, une unité McFarland correspond à environ $3.10^8$ bactéries/ml, une dilution de $1 \times 10^{-6}$ McF correspond donc à environ $3.10^2$ bactéries/ml. Par exemple quantitativement de l'ordre de 60 bactéries pour $200\mu$l, par exemple utilisés pour ensemencer des puits de microplaques dans le procédé décrit.

Selon l'invention, par « fort producteur de biofilm » on entend un microorganisme capable de synthétiser une quantité de biofilm suffisante pour empêcher l'agrégation des particules lors de l'application du champ, après maintien du milieu de culture pendant un temps donné. Il peut s'agir par exemple d'un microorganisme capable de synthétiser une quantité de biofilm suffisante pour empêcher l'agrégation des particules, à partir d'un inoculum de l'ordre de 60 à 6000 bactéries, considéré comme très faible par l'homme du métier.

Selon l'invention, par «producteur intermédiaire de biofilm » on entend un microorganisme capable de synthétiser une quantité de biofilm suffisante pour empêcher l'agrégation des particules lors de l'application du champ, après maintien du milieu de culture, pendant un temps donné après maintien du milieu de culture, à partir d'un inoculum de l'ordre de 6000 ($6.10^3$) à $6.10^5$ bactéries, considéré standard par l'homme du métier.

Selon l'invention, par « faible producteur de biofilm » on entend un microorganisme capable de synthétiser une quantité de biofilm suffisante pour empêcher l'agrégation des particules après maintien du milieu de culture, pendant un temps donné, à partir d'un inoculum supérieur à $6 \times 10^7$ bactéries, considéré fort par l'homme du métier.

En d'autres termes, un microorganisme « fort producteur de biofilm » est un microorganisme qui après culture à partir d'une faible concentration de microorganismes, par exemple comprise de $1 \times 10^{-6}$ à $1 \times 10^{-4}$ McF, empêche/entrave le mouvement des particules lors de la mise en œuvre du procédé empêchant leur regroupement/agrégation, notamment sous la forme d'un spot.

En d'autres termes, un microorganisme « producteur intermédiaire de biofilm » est un microorganisme qui après culture à partir d'une concentration intermédiaire de microorganismes, par exemple comprise supérieure à $1 \times 10^{-4}$ et inférieure à $1 \times 10^{-2}$ McF, empêche/entrave le mouvement des particules lors de la mise en œuvre du procédé empêchant leur regroupement/agrégation, notamment sous la forme d'un spot.

En d'autres termes, un microorganisme « faible producteur de biofilm » est un microorganisme qui après culture à partir d'une forte concentration de microorganismes, par exemple à partir d'une concentration supérieure ou égale à $1 \times 10^{-2}$ McF, empêche/entrave le mouvement des particules lors de la mise en œuvre du procédé empêchant leur regroupement/agrégation, notamment sous la forme d'un spot.

En d'autres termes, un microorganisme « non producteur de biofilm » est un microorganisme qui après culture quelque soit la concentration de microorganismes n'a aucun effet sur le mouvement des particules lors de la mise en œuvre du procédé et permet une agrégation des particules.

Selon l'invention le maintien des milieux de culture ensemencés peut être réalisé pendant 1 à 24 heures, de préférence 1 à 8 heures, de préférence 4 à 6 heures. L'homme du métier de part ces connaissances générales saura adapter le temps de maintien en fonction du microorganisme.

Avantageusement, le maintien des milieux de culture ensemencés peut être réalisé de 4 à 6 heures.

Avantageusement, le maintien des milieux de culture pendant 4 à 6 heures permet lors de la mise en œuvre du procédé de l'invention de conclure quant à la capacité de production du microorganisme et/ou de sa classification selon l'invention.

Avantageusement, les inventeurs ont démontré que le maintien des milieux de culture à l'étape c) pendant 4 à 6 heures permet avantageusement lorsque le microorganisme a été ensemencé à une concentration comprise de de $1 \times 10^{-1}$ à $1 \times 10^{-6}$ McF de conclure quant à la capacité de production du microorganisme et/ou de sa classification selon l'invention.

Selon l'invention, le maintien des milieux de culture ensemencés peut être réalisé pendant 5 à 12 temps de génération du microorganisme, de préférence de 6 à 10 temps de génération du microorganisme.

L'homme du métier, de par ces connaissances générales, connait le temps de génération, c'est-à-dire le temps de doublement d'une population de microorganismes. Par exemple, il peut s'agir du temps mentionné dans le document « Microbiochimie et alimentation », Alain Branger, Educagri Editions, 2012.Par exemple le temps de génération d'une bactérie peut être en moyenne de 30 minutes.

Selon l'invention, le contenant de culture peut être tout contenant de culture connu de l'homme du métier et/ou disponible dans le commerce adapté. Il peut s'agir par exemple d'un puit d'une plaque 96 puits et/ou de tout réacteur de culture connu de l'homme du métier. Préférentiellement, le contenant de culture peut être constitué par un matériau favorable à l'adhésion des microorganismes, par exemple en polystyrène (Thermo Scientific Technical Bulletin B06a Principles in adsorption to polystyrene, 2010), peut être modifié afin de favoriser son adhésion, par exemple par irradiation du contenant de culture, par exemple par irradiation d'un contenant en polystyrène (Biofiles, issue3.8, 21-24).

Le contenant de culture peut être avantageusement un contenant plan et/ou à fond plat. Avantageusement lorsque le contenant de culture est plan et/ou à fond plat, l'agrégation des particules est réalisée sur le plan et/ou le fond plat.

Selon l'invention, le procédé de l'invention peut être réalisé dans une pluralité de contenants de culture. Par exemple, le procédé de l'invention peut être réalisé dans au moins 6 contenants de culture, par exemple des puits de microplaques 96 puits, ou d'un autre format.

Il peut s'agir par exemple de contenant de culture en polystyrène en polypropylène, en polycarbonate ou en verre.

L'homme du métier de par ses connaissances générales saura adapter/choisir le contenant en fonction du microorganisme.

Selon l'invention, lorsque le procédé comprend 6 contenants de culture, il peut comprendre indépendamment dans chaque contenant un milieu de culture identique comprenant différente concentration de microorganismes. Par exemple, lesdits contenant peuvent comprendre respectivement une concentration de microorganisme de $1 \times 10^{-6}$ McF, $1 \times 10^{-5}$ McF, $1 \times 10^{-4}$ McF, $1 \times 10^{-3}$ McF, $1 \times 10^{-2}$ McF et $1 \times 10^{-1}$ McF. Un contenant additionnel peut également être ajouté, contenant tous les constituants du milieu, à l'exception du microorganisme, représentant ainsi un contrôle d'agrégation des particules, ou référence.

Selon l'invention par milieu de culture liquide on entend tout milieu de culture liquide connu de l'homme du métier et/ou disponible dans le commerce dans lequel au moins un microorganisme est susceptible de se développer. Il peut s'agir par exemple d'un milieu naturel ou synthétique. Il peut s'agir par exemple de BHI (Brain Heart Infusion ; Bouillon Cœur Cerveau), de milieu Mueller-Hinton, de bouillon glucosé. L'homme du métier de part ces connaissances générales saura choisir le milieu de culture adapté en fonction du microorganisme.

Selon l'invention le microorganisme peut être tout microorganisme connu de l'homme du métier. Il peut s'agir d'une cellule procaryote, par exemple d'une bactérie ou d'une cellule eucaryote, par exemple un champignon, une levure. Il peut s'agir par exemple de *Candida, Cryptococcus, Malassezia, Pityrosporum, Pneumocystis, Epidermophyton, Microsporum, Trichophyton.* Il peut s'agir également de protozoaires, par exemple *Entamoeba histolytica, Acanthamoeba castellanii, Naegleria fowleri.*

Parmi les cellules procaryotes, il peut s'agir par exemple de toute bactérie connu de l'homme du métier, par exemple les bactéries comprises dans le groupe, sans être limité à celui-ci, constitué de Acetobacter aurantius, Actinobacillus actinomycetemcomitans, Agrobacterium tumefaciens, Azorhizobium caulinodans, Azotobacter vinelandii, Bacillus anthracis, Bacillus brevis, Bacillus cereus, Bacillus fusiformis, Bacillus licheniformis, Bacillus megaterium, Bacillus stearothermophilus, Bacillus subtilis, Bacteroides gingivalis, Bacteroides melaninogenicus, Bartonella henselae, Bartonella quintana, Bordetella bronchiseptica, Bordetella pertussis, Borrelia burgdorferi, Branhamella catarrhalis, Brucella abortus, Brucella melitensis, Brucella suis, Burkholderia mallei, Burkholderia pseudomallei Calymmatobacterium granulomatis, Campylobacter coli, Campylobacter jejuni, Campylobacter pylori, Chlamydia pneumoniae, Chlamydia psittaci, Chlamydia trachomatis, Chlamydophila pneumoniae, Chlamydophila psittaci, Clostridium botulinum, Clostridium difficile, Clostridium perfringens, Clostridium tetani, Clostridium welchii, Corynebacterium diphtheriae, Corynebacterium fusiforme, Coxiella burnetii Ehrlichia chaffeensis, Enterococcus avium, Enterococcus durans, Enterococcus faecalis, Enterococcus faecium, Enterococcus galllinarum, Enterococcus maloratus, Escherichia coli, Francisella tularensis, Fusobacterium nucleatum, Gardnerella vaginalis Haemophilus ducreyi, Haemophilus influenzae, Haemophilus parainfluenzae, Haemophilus pertussis, Haemophilus vaginalis, Helicobacter pylori, Klebsiella pneumoniae, Klebsiella rhinoscleromatisklebsiella oxytoca, Lactobacillus acidophilus, Lactobacillus casei, Lactococcus lactis, Legionella pneumophila, Methanobacterium extroquens, Microbacterium multiforme, Micrococcus luteus, Mycobacterium avium, Mycobacterium bovis, Mycobacterium diphtheriae, Mycobacterium intracellulare, Mycobacterium leprae, Mycobacterium lepraemurium, Mycobacterium phlei, Mycobacterium smegmatis, Mycobacterium tuberculosis, Mycoplasma fermentans, Mycoplasma genitalium, Mycoplasma hominis, Mycoplasma pneumoniae Neisseria gonorrhoeae, Neisseria meningitidis, Nocardia asteroides Pasteurella multocida, Pasteurella tularensis, Porphyromonas gingivalis, Pseudomonas aeruginosa, Pseudomonas maltophilia, Rhizobium radiobacter, Rickettsia prowazekii, Rickettsia mooseri, Rickettsia psittaci, Rickettsia quintana, Rickettsia rickettsii, Rickettsia trachomae, Rochalimaea henselae, Rochalimaea quintana, Rothia dentocariosa, Salmonella enteritidis, Salmonella typhi, Salmonella typhimurium, Serratia marcescens, Shigella dysenteriae, Staphylococcus aureus, Staphylococcus epidermidis, Streptococcus agalactiae, Streptococcus avium, Streptococcus bovis, Streptococcus cricetus, Streptococcus faceium, Streptococcus faecalis, Streptococcus ferus, Streptococcus gallinarum, Streptococcus lactis, Streptococcus mitior, Streptococcus mitis, Streptococcus mutans, Streptococcus oralis, Streptococcus pneumoniae, Streptococcus pyogenes, Streptococcus rattus, Streptococcus salivarius, Streptococcus sanguis, Streptococcus sobrinus, Treponema pallidum, Vibrio cholerae, Vibrio comma, Vibrio parahemolyticus, Vibrio vulnificus, Xanthomonas maltophilia Yersinia enterocolitica,Yersinia pestis et Yersinia pseudotuberculosis, etc.

Selon l'invention dans le cas d'utilisation de bactéries anaérobies, les conditions de culture anaérobie du microorganisme pourront être obtenues par obturation de l'extrémité ouverte du réacteur de culture par exemple à l'aide de parafilm, d'un bouchon etc. ou en mettant ledit réacteur dans des conditions permettant le développement des bactéries anaérobies. Il peut s'agir par exemple de jarres d'anaérobiose, sachets générant une atmosphère appauvrie en oxygène et enrichie en dioxyde de carbone, enceintes anaérobies avec atmosphère contrôlée et/ou tout moyen connu de l'homme du métier adapté.

Selon l'invention, lesdites, au moins deux, particules, peuvent être choisies dans le groupe comprenant une particule chargée électriquement, une particule magnétique, une particule revêtue d'au moins une couche magnétique, une particule magnétisable, une particule revêtue d'une couche magnétisable, ou un mélange de deux ou plusieurs de ces particules. En fait, il peut s'agir de toute particule permettant de mettre en œuvre la présente invention.

Selon l'invention, les particules peuvent reposer sur une surface immergée dans le milieu de culture. Lesdites particules sont en position stables, c'est-à-dire au repos, en l'absence du champ magnétique, ou électrique, ou électromagnétique. Avantageusement, lesdites particules peuvent être des particules de toute forme adaptée à la mise en œuvre de la présente invention, par exemple sous forme de bille, de palet, de forme géométrique asymétrique, par exemple avec une face plane, etc.

Toute taille appropriée de particule magnétique ou magnétisable peut être utilisée. La taille peut être choisie par exemple en fonction de la taille du microorganisme, et/ou de la taille du compartiment contenant le milieu de culture pour la mise en œuvre du procédé de l'invention et/ou du microorganisme. La taille peut être par exemple de taille sensiblement identique à la taille du microorganisme générant le biofilm de manière à ce que les, au moins deux, particules puissent être incorporées dans un biofilm, par exemple formé par ledit microorganisme, l'objectif étant d'utiliser les particules magnétiques ou magnétisables comme un équivalent inerte du microorganisme. Quand ces particules sont mobiles, les microorganismes de même taille peuvent aussi se mouvoir. Quand ces particules sont immobilisées, les microorganismes de même taille ne peuvent pas non plus se déplacer. D'autre part, des particules de tailles variées peuvent permettre une analyse plus fine de la structure du biofilm : dimension des espaces au sein de la structure, organisation tridimensionnelle, stabilité de l'adhérence à la surface, par exemple au fond du tube ou de la cupule de la plaque de microdilution, dans laquelle le test est réalisé. En effet, les biofilms tendent à se détacher par lambeaux (squames) de leur support en vieillissant. Dans ce cas, on observe alors une première phase d'immobilisation des, au moins deux, particules lors du développement du biofilm. Puis une phase de dégénérescence du biofilm avec libération des, au moins deux, particules.

Par exemple les particules magnétiques ou magnétisables peuvent présenter une taille de, par exemple de 10nm à 100$\mu$m, par exemple de 0,1 à 10$\mu$m (taille des microorganismes les plus courants).

Lorsque les particules sont incorporées dans un biofilm, notamment dans la matrice complexe générée par la croissance des microorganismes, composée de corps cellulaires, des systèmes d'adhésion desdits microorganismes, par exemple des filaments, pili, fimbriae, etc., de substances plus ou moins visqueuses, par exemple des polysaccharides, alginates, etc. sécrétées par lesdits microorganismes, l'application du champ magnétique, ou électrique, ou électromagnétique ne permet pas de provoquer de mouvement desdites particules incorporées dans ledit biofilm.

Lorsque les particules ne sont pas incorporées dans ledit biofilm, par exemple lorsque la mobilité des particules n'est pas entravée par la présence de corps de microorganismes dont la taille est voisine de celles des particules, par exemple de l'ordre un à plusieurs micromètres, par exemple lorsque la mobilité des particules n'est pas entravée par la présence des systèmes d'adhésion desdits microorganismes, par exemple les filaments, pili, fimbriae, etc. générant un enchevêtrement bloquant la progression des particules, par exemple lorsque la mobilité des particules n'est pas entravée par la présence de substances plus ou moins visqueuses sécrétées par lesdits microorganismes, par exemple des polysaccharides, alginates, etc., par exemple lorsque la mobilité des particules n'est pas entravée par des molécules liées au développement du biofilm de microorganismes, l'application du champ magnétique, ou électrique, ou électromagnétique provoque le mouvement desdites particules.

Selon l'invention, le procédé de l'invention peut être mis en œuvre avec une pluralité de particules, par exemple de 2 à 10 000 000, de 1000 à 1 000 000, de de 10 000 à 1 000 000, de 100 000 à 1 000 000, de 10 000 à 100 000. La pluralité de particules permet avantageusement de détecter directement, sans dispositif complexe de visualisation et sans colorant, l'interaction entre lesdites substances au contraire des procédés de l'art antérieur utilisant une seule particule et nécessitant pour la détection de l'interaction des dispositifs complexes de visualisation ou des colorants.

Selon l'invention, les particules peuvent être éclairées, par exemple au moyen d'une source lumineuse. L'éclairage permet avantageusement d'augmenter le contraste entre les particules et la solution.

Selon l'invention, l'observation de peut être réalisée par tout moyen connue de l'homme du métier. Il peut s'agir par exemple d'un dispositif optique, par exemple un microscope, un appareil photo, d'un scanner de document, par exemple un scanner EPSON Perfection V600 Photo, une observation visuelle.

Selon l'invention, l'observation peut permettre de mesurer, par exemple l'intensité, le contraste, la variance, d'image, par exemple via tout moyen connu de l'homme du métier, par exemple un logiciel d'imagerie, par exemple ImageJ permettant, par exemple de mesurer, par exemple des différences de contrastes, d'intensités, correspondant par exemple aux particules, dans des zones d'une image à une autre et ainsi de déterminer les différences d'une observation à une autre. Il peut s'agir, par exemple de comparer les images obtenues avant et après application de l'action mécanique, hydrodynamique ou physique, par exemple en faisant une soustraction entre des images, par exemple en mesurant le coefficient de corrélation entre les images.

Selon l'invention, l'utilisation de particules émettrices d'un signal, par exemple des particules colorées, excitables, fluorescentes, phosphorescentes, luminescentes, radioactives peut permettre, par exemple une observation automatisée

Selon l'invention, les conditions de réalisation du procédé de l'invention, en particulier de culture du microorganisme, sont des conditions de cultures standardisées, par exemple le pH, la température, l'oxygénation, etc.

Selon l'invention le maintien des milieux de culture ensemencés peut être réalisé à une température comprise de 10 à 45 °C, de préférence de 25°C à 42°C, par exemple égale à 37°C

Selon l'invention le maintien des milieux de culture ensemencés peut être réalisé sous une atmosphère naturelle, dans un incubateur classique par exemple, ou sous atmosphère contrôlée pour les microorganismes microanaérophiles ou anaérobies stricts, selon tout moyen connu de l'homme du métier

Selon l'invention, le champ peut être un champ magnétique, ou électrique ou électromagnétique.

Selon l'invention l'application du champ magnétique peut être réalisé par tout moyen connu de l'homme du métier et/ou disponible dans le commerce et/ou adapté.

Selon l'invention, le champ magnétique, ou électrique ou électromagnétique peut être tout champ permettant de mettre en mouvement lesdites, au moins deux particules sur ladite surface immergée dans ladite solution, par exemple un champ électromagnétique ou un champ magnétique. Le champ magnétique, ou électrique ou électromagnétique peut être généré, par exemple par un aimant ou par un solénoïde. L'aimant peut être par exemple sous forme de barreau, de pointe, de pièce, etc. ou toute forme appropriée pour la mise en œuvre de la présente invention. Le champ peut, par exemple être appliqué par tout moyen connu de l'homme du métier, par exemple par impulsion, par augmentation progressive du champ électromagnétique, par variations de champ électromagnétique ou par une combinaison ces applications.

Selon l'invention, le procédé peut comprendre en outre une étape e') préalable/postérieure ou remplaçant l'étape e) de détermination de la valeur de l'Index Biofilm (BFI) pour chacun des milieux ensemencés et calcul de l'indice de formation potentielle du biofilm par contenant (BPc) selon la formule (I) suivante :

$$BPc= [1-(BFIe / BFIn)] \ (I)$$

dans laquelle BFIe correspond à la valeur de l'index biofilm dans le milieu ensemencé et BFIn correspond à la valeur de l'index biofilm dans un contenant ne comprenant pas de microorganismes.

Selon l'invention, la valeur de BFIn correspond au contrôle négatif.

Selon l'invention , la formation de biofilm peut être identifiée lorsque la valeur de BPc est supérieure à une valeur Seuil (S). Selon l'invention, la valeur seuil (S) peut être calculée selon la formule (II) suivante

$$S= 1 - [(mBFIn - 3 \ X \ stmBFIn)/2] \ / \ mBFIn \ (II)$$

dans laquelle mBFIn est égale à la moyenne des BFI des contrôles négatifs, stmBFIn est également à la déviation standard de la moyenne des BFI des contrôles négatifs calculée.

Selon l'invention, le procédé peut comprendre en outre une étape e") postérieure ou simultanée à l'étape e') de détermination de la valeur seuil (S) selon la formule (II) suivante :

$$S= 1 − [(mBFIn − 3 \times stmBFIn)/2] / mBFIn \text{ (II)}$$

dans laquelle mBFIn est égale à la moyenne des BFI des contrôles négatifs, stmBFIn est également à la déviation standard de la moyenne des BFI des contrôles négatifs calculée.

L'homme du métier de part ces connaissances générales saura calculer la moyenne des BFI des contrôles négatifs ainsi que la déviation standard de la moyenne.

Dans la présente, le procédé de l'invention peut comprendre pour chaque mesure au moins trois contrôles négatifs à partir desquels la valeur mBFIn et la valeur stmBFIn sont calculées. Par exemple, pour chaque calcul de BPc, le procédé peut comprendre au moins trois valeurs de BFIn à partir desquels la moyenne des BFI des contrôles négatifs (mBFIn) ainsi que la déviation standard de la moyenne (stmBFIn) peuvent être calculées.

L'homme du métier de par ses connaissances générales saura adapter le nombre de contrôle négatif pour le calcul de la moyenne des BFI des contrôles négatifs (mBFIn) ainsi que la déviation standard de la moyenne (stmBFIn) peuvent être calculées.

Selon l'invention, lorsque plusieurs contrôles négatifs sont utilisés pour calculer la valeur de mBFIn dans la formule (II), la valeur de BFIn dans la formule (I) peut être égale à la valeur de mBFIn.

Selon l'invention, les inventeurs ont démontré de manière surprenante que la capacité de production de biofilm peut être déterminée comme suit :

- une valeur de BPc supérieure ou égale à la valeur seuil S à partir d'une concentration de microorganismes comprise de $1 \times 10^{-6}$ à $1 \times 10^{-4}$ McF correspondant à un microorganisme fort producteur de biofilm,
- une valeur de BPc supérieure ou égale à la valeur seuil S à partir d'une concentration de microorganismes supérieure à $1 \times 10^{-4}$ et inférieure à $1 \times 10^{-2}$ McF correspondant à un microorganisme producteur intermédiaire de biofilm,
- une valeur de BPc supérieure ou égale à la valeur seuil S à partir d'une concentration de microorganismes de $1 \times 10^{-2}$ McF correspondant à un microorganisme faible producteur de biofilm,
- une valeur de BPc inférieure à la valeur seuil S quelle que soit la concentration correspondant à un microorganisme non producteur de biofilm.

Dans la présente l'Index Biofilm (BFI) est obtenu par analyse des composantes de couleurs rouge vert bleu des pixels de l'image afin de déterminer la densité de particules par pixel, puis par calcul de la corrélation entre les particules pour déterminer la densité d'agrégation des particules exprimée par une valeur BFI. L'index BFI est décrit notamment dans le document Chavant et al. 2007 [5].

**Brève description des figures** :

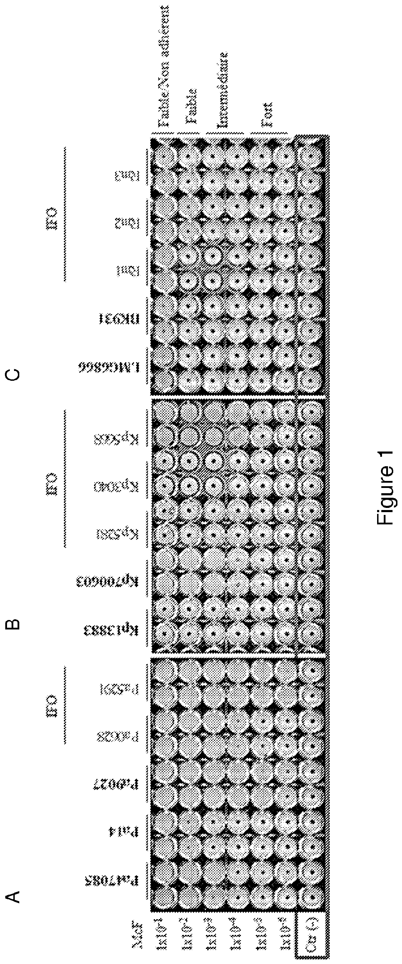

La Figure 1 est une photographie de plaque 96 puits. Elle représente la formation de biofilm sur des plaques polystyrène 96 puits par des bactéries à Gram négatif: A) P. aeruginosa (Pa), B) K. pneumonie (Kp) et C) R. mannitolilytica isolée de l'hôpital IFO. Les images ont été obtenues après aimantation des plaques sur le block test et la numérisation avec le lecteur de plaque. Les souches de laboratoire de référence sont indiquées en gras. L'absence de formation de biofilm est révélée par la présence du point noir central dans les puits correspondant au regroupement des particules. La présence de biofilm est révélée par l'absence de point noir au centre des puits. Les puits correspondant aux contrôles négatifs (Ctr (-)) contenant seulement du milieu BHI et les microparticules magnétiques correspondent à la ligne inférieure de puits.

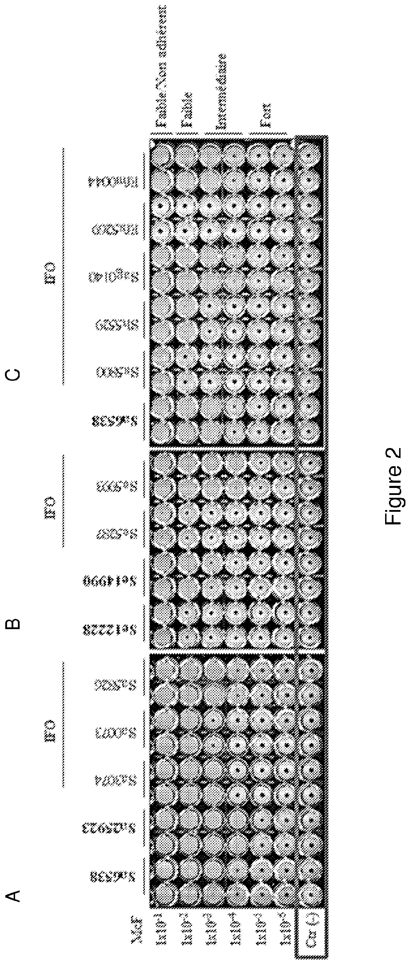

La Figure 2 est une photographie de plaque 96 puits. Elle représente la formation de biofilm de formation de biofilm sur des plaques polystyrène 96 puits pour les bactéries à Gram positif: A) S. aureus (SA), B) S. epidermidis (SE) et C) différentes bactéries à Gram positif isolées à l'hôpital IFO. Les images ont été obtenues après aimantation des plaques sur le Block test et la numérisation avec un lecteur de plaque. Les souches de laboratoire de référence sont indiquées en gras. L'absence de formation de biofilm est révélée par la présence du point noir central dans les puits correspondant au regroupement des particules. La présence de biofilm est révélée par l'absence de point noir au centre des puits. Les puits correspondant aux contrôles négatifs (Ctr (-)) contenant seulement du milieu BHI et les microparticules magnétiques sont entourés en rouge.

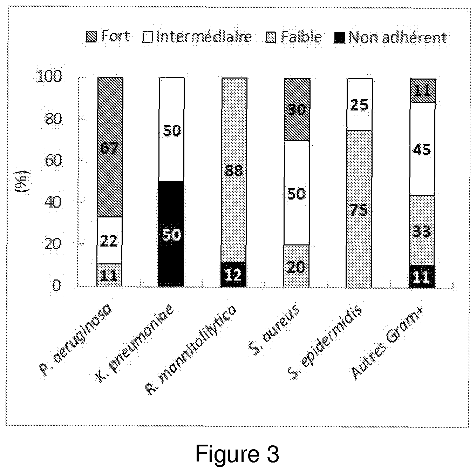

La Figure 3 est un diagramme en bâton représentant le pourcentage d'isolats cliniques de chaque espèce classés en fonction de leur capacité de la production de biofilm mesurée par le procédé à savoir fort producteur (zone gris foncé), producteur intermédiaire (zone blanche), faible producteur (zone gris claire) et non producteur/non adhérent (zone en noir).

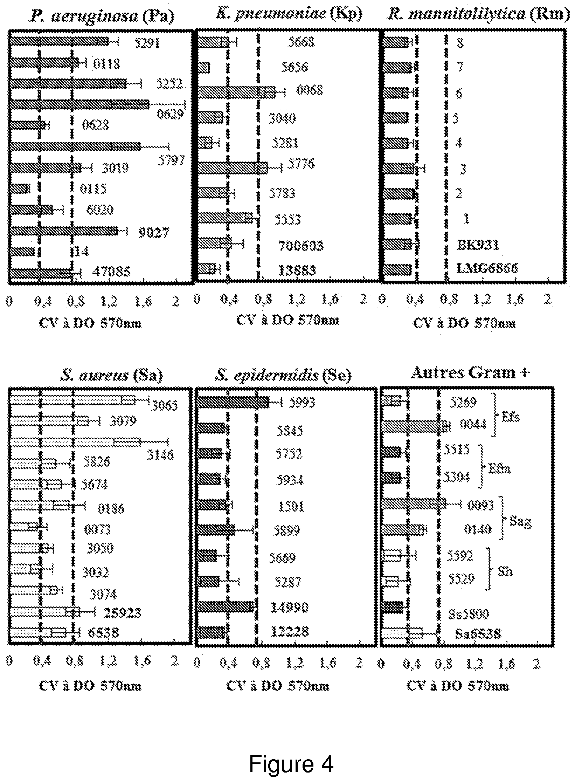

La Figure 4 est un diagramme en bâton représentant les résultats de l'analyse quantitative de la formation de biofilm par coloration au Cristal Violet (CV) d'isolats cliniques de P. aeruginosa, K. pneumoniae, R. mannitolilytica, S. aureus, S epidermidis et d'autres bactéries à Gram positif. Les isolats ont été classés selon leurs valeurs seuil lors de la mesure de la densité optique (DO) à une longueur de 570nm (DO570) du milieu. Lorsque la valeur de la DO570 était <0,18 les isolats sont non producteur/non adhérent, faible producteur quand la DO570 est comprise entre 0,18 et 0,37; producteur modéré/intermédiaire quand la DO570 est comprise entre 0,37 et 0,74; fort ou haut producteur lorsque la DO570 est supérieure à 0,74. Les barres d'erreur indiquent l'erreur standard. Lignes en pointillés (- - -) indique les valeurs seuils (cut-off) aux DO570 <0,37 et à DO570 <0,74.

D'autres avantages pourront encore apparaître à l'homme du métier à la lecture des exemples ci-dessous, illustrés par les figures annexées, donnés à titre illustratif.

## EXEMPLES

**Exemple 1 : Evaluation de la capacité de production de biofilm de microorganisme**

Dans l'exemple ci-dessous, un exemple de procédé selon l'invention est notamment désigné procédé de caractérisation ou procédé cBFRT. Dans cet exemple les matériaux et méthodes utilisées étaient les suivant :

Souches et conditions de croissance

Souche de référence : Staphylococcus aureus American Type Culture Collection (ATCC) 25923, Staphylococcus aureus ATCC 6538, Staphylococcus epidermidis ATCC 14990, Staphylococcus epidermidis ATCC 12228, Klebsiella pneumoniae ATCC 700603, Klebsiella pneumoniae ATCC 13883, Pseudomonas aeruginosa ATCC 47085, Pseudomonas aeruginosa ATCC 9027, Pseudomonas aeruginosa PA14, Ralstonia mannitolilytica LMG 6866, Ralstonia mannitolilytica BK931 et les isolats cliniques de bactéries ont été cultivées en aérobie sur une gélose non sélective (gélose au sang, gélose chocolat, agar McConkey) (Oxoid, Hampshire, Royaume-Uni) à 37 ° C.

Sélection des isolats

Un total de 52 isolats cliniques recueillis auprès de patients atteints d'infections nosocomiales admis à l'hôpital IFO de Rome ont été évalués. Les bactéries ont été collectées à partir de différents matériaux, y compris d'infections chroniques (ulcères), de cathéters intraveineux et des voies urinaires, du sang, de l'urine, de crachats et d'échantillons de lavage nasobronchial. Un ulcère a été classé comme chronique s'il existait depuis au moins 3 mois (Dissemond, 2006). L'étude comprenait également des souches présentant une résistance accrue aux antibiotiques couramment utilisés comme déterminé selon la norme des tests de sensibilité aux antibiotiques par le système de VITEK2 (bioMérieux, Florence, Italie). En particulier, les souches de P. aeruginosa résistantes à trois, ou plus, classes d'antibiotiques ont été considérés comme des microorganismes multi-résistants (MDR). Les souches K. pneumoniae résistant à la plupart des antibiotiques bêta-lactamines, y compris les pénicillines, céphalosporines et l'aztréonam monobactame se développant sur milieu sélectif chromogène chromID ESBL (bioMérieux, Florence, Italie) ont été classées à spectre étendu de bêta-lactamase (ESBL). La détection phénotypique des producteurs ESBL a encore été définie à l'aide du test de disque (Oxoid, Royaume-Uni), comme décrit dans De Gheldre, 2003; Moremi, 2014. Les souches K. pneumoniae résistantes aux carbapénèmes et identifiées par un milieu sélectif chromogène chromID CARBA (bioMérieux, Florence, Italie) sont référencés comme K. pneumoniae produisant des carbapénémases (KPC). Les Staphylococcus aureus résistants à la méticilline (MRSA) étaient productrices de la protéine de liaison à la pénicilline (PBP) après confirmation par le test d'agglutination latex PBP2 'kit (OXOID Ltd, Basingstoke, Royaume-Uni). Les Entérocoques résistant à la vancomycine (VRE) ont été identifiés par des tests de sensibilité et confirmé par des milieux sélectifs chromogènes chromID VRE (bioMérieux, Florence, Italie). Les taux de sensibilité aux antibiotiques ont été comparés avec ceux définis par la concentration minimale inhibitrice (CMI) selon les critères d'interprétation recommandée par le Comité Européen sur les Tests de Sensibilité aux Antimicrobiens (EUCAST). Le nombre d'espèces et les caractéristiques des souches testées sont signalés dans le tableau 2. Les isolats ont été stockés à -70°C dans des tubes de CRYOBANK (Copan Italia SpA) et cultivés pendant la nuit à 37 ° C sur une boite de gélose spécifique avant le test.

Préparation de l'inoculum

La formation de biofilm a été évaluée par le BioFilm Ring Test (BFRT) (BioFilm Control, Saint-Beauzire, France) en utilisant le kit disponible dans le commerce (BioFilm Control, Saint Beauzire, France). La solution de toner (TON004) contenant des billes magnétiques a été mélangée dans un milieu Brain Heart Infusion (BHI, Difco, Detroit, MI, USA) selon les instructions du fabricant. Pour la détermination de la capacité de production de biofilm/classification des microorganismes l'inoculum a été préparé comme suit. Une culture fraîche de bactéries sur gélose d'une nuit a été utilisée pour chaque souche à tester. Les bactéries cultivées ont ensuite été transférées par une anse d'inoculation stérile dans un tube stérile contenant 2 ml de solution saline à 0,45% à l'équivalent de 1,0 $\pm$ 0,3 standard de turbidité McFarland (McF) et bien mélangées. Ensuite, 200 $\mu$l de l'inoculum ont été transférés dans les puits d'une plaque de polystyrène à 96 puits. De cet inoculum initial, des dilutions sérielles d'un facteur 10 ont été réalisées, de $1 \times 10^{-1}$ à $1 \times 10^{-6}$ McF en transférant 20 $\mu$l de la suspension microbienne dans 200 $\mu$l du mélange BHI / TON.

Une ou plusieurs souches de laboratoire ont été incluse(s) dans chaque plaque comme référence standard et contrôle de la qualité. Un puits contenant le BHI / TON mix sans cellules microbiennes a été utilisée comme contrôle négatif dans chaque expérience.

Après cinq heures d'incubation à 37°C sans agitation (culture statique), les puits ont été recouverts de quelques gouttes de liquide de contraste (huile inerte opaque utilisé pour l'étape de lecture, incluse dans le kit BFRT) placé pendant 1 min sur le bloc portant 96 mini-aimants (bloc test) et scanné avec un lecteur de plaque spécialement conçue (Pack BIOFILM, BioFilm Control, Saint Beauzire, France). La force d'adhérence de chaque souche a été exprimée en indice BioFilm (BFI), qui a été calculé par un logiciel spécialisé initialement décrit dans Chavant, 2007. Les valeurs de BFI ont été utilisées pour mesurer le potentiel de formation de biofilm (BP) en utilisant la formule:

$$BPc = [1-(BFIe / BFIn)] \ (I)$$

dans laquelle BFIe correspond à la valeur de l'index biofilm dans le milieu ensemencé et BFIn correspond à la valeur de l'index biofilm dans un contenant ne comprenant pas de microorganismes.

La valeur seuil (S) a été calculée selon la formule (II) suivante

$$S = 1 - [(mBFIn - 3 \ X \ stmBFIn)/2] / mBFIn \ (II)$$

dans laquelle mBFIn est égale à la moyenne des BFI des contrôles négatifs, stmBFIn est également à la déviation standard de la moyenne des BFI des contrôles négatifs calculée.

Dans cet exemple, les valeurs de BFIn étaient comprises de 18 à 20. La valeur de S calculée était égale à 0,53.

Les valeurs de BPc au-dessus de la valeur S calculée ont été considérées comme des producteurs de biofilm. Ainsi, la dernière dilution pour laquelle la valeur est au-dessus de la valeur S calculée a permis identifier la capacité du micro-organisme pour former un biofilm. En conséquence, les micro-organismes ont été classés dans les catégories suivantes: cellules non adhérentes, producteurs faibles, modérés et forts de biofilm. Chaque culture microbienne a été analysée en double exemplaire, et les expériences ont été répétées au moins 3 fois pour chaque souche afin d'évaluer la reproductibilité, l'exactitude et la précision de la mesure. Les valeurs ont été considérées comme valables si l'écart type entre les doublons était inférieur à 8%. Les réplicas ont montré une cohérence complète dans le classement/catégorisation des microorganismes.

Evaluation de la formation de biofilm avec le test au Cristal Violet

Des plaques de polystyrène à 96 puits stériles ont été inoculées avec 200 $\mu$l d'une suspension bactérienne initiale ($10^5$ CFU / ml) dans du milieu BHI et incubé à 37°C pendant 24 et 48 heures sans agitation. Chaque condition a été réalisée en triple. Le milieu a été retiré des puits, qui ont été lavés 3 fois avec 200 $\mu$l d'eau distillée stérile. Les plaques ont été séchées à l'air pendant 45 minutes et les cellules adhérentes colorées avec 200 $\mu$l de solution de Cristal Violet à 0,1%. Après 20 minutes, le colorant a été éliminé et les puits lavés quatre fois avec 300 $\mu$l d'eau distillée stérile pour éliminer l'excès de colorant. Le colorant incorporé par les cellules formant un biofilm a été dissous avec 200 $\mu$l d'un mélange 80/20% éthanol/acétone et l'absorbance de chaque puits a été mesurée par spectrophotométrie à 570 nm (DO$_{570}$) en utilisant le Système PhD Ix ™ automatisé (Bio-Rad Laboratories, Hercules, CA, USA).

Pour l'analyse comparative des valeurs de DO$_{570}$ ont été utilisés pour classer semi-quantitativement la production de biofilm pour les souches bactériennes selon le procédé décrit dans Stepanovic et al (Stepanovic, 2000). En bref, la Densité Optique seuil (ODC) a été définie comme trois écarts-types au-dessus de la Densité Optique (DO) moyenne du contrôle négatif. Aussi, les souches ont été classées comme suit: DO <ODc = non adhérentes; ODc < DO < 2 × ODc = faible producteur de biofilm; 2 × ODc < DO < 4 × ODc = producteur modéré de biofilm; et DO > 4 × ODc = fort producteur de biofilm.

Tous les dosages ont été effectués en triplicats, avec des souches de référence et des isolats cliniques testés à trois reprises pour évaluer des variations éventuelles dans les conditions de dosage du biofilm.

Méthodes statistiques

Le test de coefficient kappa a été utilisé pour déterminer la cohérence entre les résultats obtenus avec le procédé de caractérisation et le Cristal Violet (CV). La cohérence a été calculée selon Vieia (Viera, 2005): kappa = 0,81 - 1, très bonne; kappa = de 0,61 à 0,80, bonne; kappa 0,41 à 0,60, modérée; kappa = 0,21 au 0,40, équitable; kappa ≤ 0,20, faible. Les résultats de la formation de biofilm obtenus avec les deux méthodes ont été comparés en utilisant en outre le test de McNemar. Les valeurs de p <0,05 ont été considérées comme significatives.

Résultats et discussion

Normalisation des inoculums bactérienne en Standard McFarland

Etant donné que différents microorganismes, y compris des souches mucoïdes ou non mucoïdes ou autres caractéristiques, telles que la taille, peuvent affecter la mesure de la concentration cellulaire initiale et par conséquent la fiabilité de l'essai, la précision de l'inoculum initial a été mesurée avec un densitomètre et vérifiée par comptage des Unité Formant Colonie (UFC) (Welch, 2012).

Les données rapportées dans le tableau 1, ont montré qu'une unité McF variait de $0,6 × 10^9$ CFU / ml pour la R.mannitolilytica à $1,4 × 10^9$ CFU / ml pour P. aeruginosa. Ce résultat démontre que la mesure de l'inoculum initial avec le densitomètre ne génère pas de différences significatives. La valeur moyenne des CFU / ml pour les différentes souches bactériennes à partir de 1 McF correspond à $1,0 × 10^9 ± 3,6 × 10^8$ ce qui est dans la même gamme de valeur qu'indiqué précédemment pour les bactéries (Eng et al, 1984). Par conséquent, les valeurs UFC montrent que la norme McF est hautement reproductible, indépendamment des espèces bactériennes ou phénotypes microbiens, assurant la précision du dosage au moment de l'inoculum.

Tableau 1: Correspondance UFC / ml à 1McFarland pour les bactéries utilisées pour inoculer le dosage cBFRT. Les valeurs représentent la moyenne UFC / ml ± SD de deux répétitions.

| Souche microbienne | CFU/mL |
|---|---|
| *P. aeruginosa* | $1.4 × 10^9 ± 7.2 × 10^8$ |
| *K. pneumoniae* | $0.9 × 10^9 ± 7.9 × 10^8$ |
| *R. mannitolilytica* | $0.6 × 10^9 ± 2.6 × 10^8$ |
| *S. aureus* | $1.3 × 10^9 ± 8.4 × 10^8$ |
| *S. epidermidis* | $1.1 × 10^9 ± 7.1 × 10^8$ |
| Autre Gram+ | $1.1 × 10^9 ± 8,7 × 10^8$ |
| Moyenne | $1.0 × 10^9 ± 3.2 × 10^8$ |

Evaluation de la production de biofilm dans des bactéries à Gram négatif et à Gram positif

L'aptitude à former un biofilm a été initialement évaluée sur des isolats de P. aeruginosa, qui est un pathogène opportuniste à Gram négatif avec une remarquable capacité à former un biofilm, ce qui assure sa persistance dans l'environnement et dans les maladies infectieuses chroniques (Høiby et al, 2010).

Parmi les 12 souches testées, trois étaient des souches de laboratoire avec une capacité connue pour former un biofilm. Ceux-ci comprenaient respectivement Pa47085 (capacité modérée) (Schaber, 2004), PA14 (capacité faible) (Rahme, 1995), et Pa9027 (capacité forte), (Stapleton, 1993), tandis que les 9 souches de P. aeruginosa restants étaient des isolats cliniques recueillis à partir patients hospitalisés (tableau 2).

Tableau 2: Caractéristiques des isolats cliniques utilisés dans cette étude. Multi-Drogues Résistantes (MDR), à spectre étendu de bêta-lactamase (BLSE), K. pneumoniae productrices de carbapénémase (KPC), S. aureus résistante à la méthicilline (MRSA), entérocoques résistants à la vancomycine (VRE). Infection des voies urinaires associée à un cathéter - (UTI-CA), cathéter veineux central (CVC), Infection sanguine associée à un cathéter (CA-BI).

| Espèces bactériennes | Isolats cliniques | Phénotype | Site d'isolement |
|---|---|---|---|
| **Bactéries à Gram négatif** | | | |
| *P. aeruginosa* | 9 | MDR (2) | CA-BI (2) |
| | | MDR (1)<br>- | Ulcère Chronique (2)<br>Plaie (1) |
| | | - | Urine (1) |
| | | - | Respiratoire (3) |
| *K. pneumoniae* | 8 | KPC (1)<br>- | CVC (1)<br>Sang (2) |
| | | - | Plaie (1) |
| | | ESBL (1) | CA-UTI (2) |
| | | - | Respiratoire (2) |
| *R. mannitolilytica* | 8 | - | Sang (8) |
| **Bactéries à Gram positif** | | | |
| *S. aureus* | 10 | MRSA (1) | CVC (1) |
| | | MRSA (3) | Ulcère chronique (6)<br>Peau (2) |
| | | | Respiratoire (1) |
| *S. epidermidis* | 8 | - | Sang (6) |
| | | - | Plaie (2) |
| autre Gram+ | 9 | - | Sang (2) |
| | | VRE (1) | Plaie (6) |
| | | - | Urine (1) |
| espèce bactérienne | isolats cliniques | Phénotype | Site d'isolation |
| **Bactéries à Gram négatif** | | | |
| *P. aeruginosa* | 9 | MDR (2) | CA-BI (2) |
| | | MDR (1)<br>- | Ulcère Chronique (2)<br>plaie (1) |
| | | - | Urine (1) |
| | | - | respiratoire (3) |
| *K. pneumoniae* | 8 | KPC (1)<br>- | CVC (1)<br>sang (2) |
| | | - | plaie (1) |
| | | ESBL (1) | CA-UTI (2) |
| | | - | respiratoire (2) |
| *R. mannitolilytica* | 8 | - | sang (8) |

(suite)

| Bactéries à Gram positif | | | |
|---|---|---|---|
| *S. aureus* | 10 | MRSA (1) | CVC (1) |
| | | MRSA (3) | Ulcère Chronique (6) |
| | | - | peau (2) |
| | | - | respiratoire (1) |
| *S. epidermidis* | 8 | - | sang (6) |
| | | - | plaie (2) |
| autre Gram+ | 9 | - | sang (2) |
| | | VRE (1) | plaie (6) |
| | | - | Urine (1) |

Après cinq heures d'incubation, la souche de référence PA14 a immobilisé les billes magnétiques seulement à la plus forte concentration de cellules (McF = $1 \times 10^{-2}$), ce qui suggère une faible capacité d'adhérence et la qualifie comme faible productrice de biofilm (Figure 1). De même, PA14 a été classée comme faible producteur de biofilm (tableau 3A). Pa47085 adhère plus facilement à la surface des puits et bloque les billes magnétiques à une concentration de McF= $1 \times 10^{-3}$, identifiant ainsi cette souche comme producteur intermédiaire de biofilm. De même, Pa9027 a été confirmée en tant que fort producteur de biofilm à une concentration cellulaire de McF= $1 \times 10^{-6}$. Ces résultats sont cohérents avec ceux décrits précédemment pour ces souches de laboratoire (Schaber, 2004; Rahme, 1995; Stapleton, 1993).

Parmi les isolats cliniques, six souches se sont avérées être des forts producteurs de biofilm. En particulier ceci inclut les trois souches MDR, dont deux provenaient de patients atteints d'infections sanguines liées aux cathéters (Pa6020-IFO, Pa5252-IFO), d'ulcères chroniques (Pa5797-IFO), et les souches isolées à partir d'un lavage broncho-alvéolaire d'un patient atteint de mucoviscidose (Pa0629-IFO), du liquide pleural (Pa5291-IFO) et provenant d'un patient avec une plaie infectée (Pa0118-IFO). Deux souches ont été classées en tant que producteurs intermédiaires de biofilm, dérivant d'un patient souffrant d'un ulcère chronique (Pa3019-IFO) et d'un autre lavage broncho-alvéolaire d'un patient atteint de mucoviscidose (Pa0628-IFO). Inversement, la seule souche faible productrice de biofilm était une souche mucoïde isolée d'une infection urinaire (Pa0115-IFO).

Le test a ensuite été utilisé pour évaluer la capacité de Klebsiella pneumoniae, l'un des agents pathogènes nosocomiaux les plus importants (Podschun, 1998), à former un biofilm. Les résultats, résumés sur la figure 1B et dans le tableau 3B, montrent que les souches de référence Kp700603 et Kp13883 étaient respectivement non adhérente/productrice et productrice intermédiaire de biofilm ce qui confirme les rapports précédents (Naparstek, 2014). En ce qui concerne les souches cliniques, Kp0068-IFO (infection urinaire), Kp5553-IFO (une souche BLSE de sonde urinaire), Kp5668-IFO (une souche KPC à partir d'un cathéter veineux central) et Kp5776-IFO (de liquide pleural), elles ont été trouvées être des productrices intermédiaires de biofilm. Inversement, les souches isolées à partir d'hémocultures (Kp5656-IFO, Kp5281-IFO, Kp5783-IFO, Kp3040-IFO), mais ne provenant pas de bactériémies liées aux cathéters, ont montré un phénotype mucoïde et ont été jugées non-adhérentes et non productrices de biofilm. Ces données sont cohérentes avec les rapports précédents indiquant une fréquence plus élevée de souches formant un biofilm parmi les K. pneumoniae isolées à partir d'environnements physiologiques non fluides, ce qui peut contribuer à expliquer la difficulté à éradiquer ces infections une fois qu'elles sont établies dans les tissus solides (Sanchez, 2013). Une autre observation importante émergeant du nombre restreint d'isolats cliniques analysés était l'incidence relativement élevée des souches non adhérentes. Plus précisément, nous avons constaté que seulement 50% des K. pneumoniae inclus dans cette étude étaient capables de produire du biofilm. Cette valeur est en accord avec des études précédentes in vitro démontrent que seulement 40% de K. pneumoniae isolées à partir de différents matériaux, étaient en mesure de produire du biofilm (Yang, 2008).

Tableau 3 : Analyse des plaques obtenues avec le procédé de caractérisation pour A) P. aeruginosa, B) K. pneumonie et C) R. mannitolilytica.

| A) McF | Pa47085 | Pa14 | Pa9027 | Pa6020-IFO | Pa0115-IFO | Pa3019-IFO | Pa5797-IFO | Pa0628-IFO | Pa0629-IFO | Pa5252-IFO | Pa0118-IFO | Pa5291-IFO | |
|---|---|---|---|---|---|---|---|---|---|---|---|---|---|
| $1 \times 10^{-1}$ | 0.97 | 0.98 | 0.99 | 0.98 | 0.90 | 0.97 | 0.99 | 1.00 | 1.00 | 0.97 | 0.98 | 1.00 | Non-adhérent/Faible |
| $1 \times 10^{-2}$ | 0.97 | 0.72 | 0.99 | 0.96 | 0.33 | 0.99 | 1.00 | 1.00 | 1.00 | 0.95 | 0.97 | 1.00 | Faible |
| $1 \times 10^{-3}$ | 0.96 | 0.01 | 0.95 | 0.92 | 0.12 | 0.98 | 0.99 | 0.98 | 0.98 | 0.98 | 0.93 | 1.00 | Intermédiaire |
| $1 \times 10^{-4}$ | 0.01 | -0.02 | 0.97 | 0.74 | 0.06 | 0.64 | 0.87 | 0.58 | 0.96 | 0.96 | 0.89 | 1.00 | Intermédiaire |
| $1 \times 10^{-5}$ | 0.03 | 0.03 | 0.96 | 0.57 | 0.07 | -0.02 | 0.53 | 0.07 | 0.87 | 0.92 | 0.78 | 1.00 | Fort |
| $1 \times 10^{-6}$ | 0.05 | 0.05 | 0.52 | 0.39 | 0.08 | 0.00 | 0.49 | -0.01 | 0.71 | 0.87 | 0.56 | 0.93 | Fort |
| | 1 | 2 | 3 | 4 | 5 | 6 | 7 | 8 | 9 | 10 | 11 | 12 | |

| B) McF | Kp13883 | Kp700603 | Kp5553-IFO | Kp5776-IFO | Kp5668-IFO | Kp5281-IFO | Kp3040-IFO | Kp0068-IFO | Kp5656-IFO | Kp5783-IFO | |
|---|---|---|---|---|---|---|---|---|---|---|---|
| $1 \times 10^{-1}$ | 0.07 | 0.98 | 0.92 | 0.86 | 1.00 | 0.01 | 0.04 | 1.00 | -0.01 | 0.15 | Non-adhérent/Faible |
| $1 \times 10^{-2}$ | 0.08 | 0.94 | 0.91 | 0.81 | 0.94 | 0.01 | 0.04 | 1.00 | 0.00 | 0.05 | Faible |
| $1 \times 10^{-3}$ | 0.03 | 0.82 | 0.84 | 0.76 | 0.95 | -0.02 | 0.04 | 0.99 | -0.01 | 0.00 | Intermédiaire |
| $1 \times 10^{-4}$ | 0.07 | 0.22 | 0.37 | 0.26 | 0.92 | 0.01 | 0.06 | 0.75 | 0.04 | 0.02 | Intermédiaire |
| $1 \times 10^{-5}$ | -0.01 | -0.03 | 0.05 | 0.00 | 0.25 | 0.02 | 0.00 | 0.01 | 0.04 | -0.01 | Fort |
| $1 \times 10^{-6}$ | 0.00 | 0.02 | 0.02 | 0.02 | 0.11 | 0.04 | 0.01 | -0.05 | 0.03 | 0.01 | Fort |
| | 1 | 2 | 3 | 4 | 5 | 6 | 7 | 8 | 9 | 10 | |

| C) McF | LMG 6866 | BK931 | Rm1-IFO | Rm2-IFO | Rm3-IFO | Rm4-IFO | Rm5-IFO | Rm6-IFO | Rm7-IFO | Rm8-IFO | |
|---|---|---|---|---|---|---|---|---|---|---|---|

| | 1 | 2 | 3 | 4 | 5 | 6 | 7 | 8 | 9 | 10 | |
|---|---|---|---|---|---|---|---|---|---|---|---|
| $1\times10^{-1}$ | 0.88 | 0.66 | 0.88 | 0.99 | 0.59 | 0.57 | 0.91 | 0.51 | 0.57 | 0.94 | Non-adhérent/Faible |
| $1\times10^{-2}$ | 0.26 | 0.14 | 0.17 | 0.61 | 0.13 | 0.11 | 0.16 | 0.12 | 0.15 | 0.54 | Faible |
| $1\times10^{-3}$ | 0.09 | 0.03 | 0.10 | 0.13 | 0.05 | 0.07 | 0.07 | 0.10 | 0.09 | 0.15 | Intermédiaire |
| $1\times10^{-4}$ | 0.00 | 0.04 | 0.07 | 0.01 | 0.11 | 0.12 | 0.02 | 0.10 | 0.10 | 0.04 | Intermédiaire |
| $1\times10^{-5}$ | 0.02 | 0.06 | 0.06 | 0.10 | 0.09 | 0.12 | 0.02 | 0.09 | 0.10 | 0.10 | Fort |
| $1\times10^{-6}$ | 0.03 | 0.10 | 0.05 | 0.03 | 0.09 | 0.13 | 0.01 | 0.09 | 0.11 | 0.09 | Fort |

Dans le tableau ci-dessus, les bactéries ont été classées en fonction de la valeur de BP, également dénommé BPc, mésurée. Le cut-off/seuil a été établi à 0.53. Une valeur comprise entre 1 et 0,53 correspond à une formation de biofilm, une valeur inférieur à 0,53 une absence éventuelle de biofilm, ou une formation non significative de biofilm

R. mannitolilytica est rarement isolé à partir d'échantillons cliniques et représente l'espèce la plus répandue du genre Ralstonia trouvée chez les patients atteints de mucoviscidose (Coenye, 2005). R. mannitolilytica a été associée à des épidémies dans le monde entier (Ryan, 2014) et l'hypothèse de la capacité de cette bactérie à former un biofilm a maintes fois été émise comme une stratégie pour survivre dans des environnements difficiles et dans les milieux cliniques, bien qu'elle n'ait été que rarement testée. Les souches présentes analysées étaient des isolats cliniques isogéniques d'une épidémie survenue dans le service d'oncologie dans l'hôpital IFO en 2014. Deux souches de référence, R. mannitolilytica LMG 6866 (De Baere, 2001) et R. mannitolilytica BK931 (Marroni, 2003) ont été évaluées en comparaison, bien que leur capacité de production de biofilm soit inconnue. Les résultats de la figure 1C et le tableau 3C ont révélé que les souches de référence LMG 6866 et BK931 étaient faibles productrices de biofilm. De manière similaire, les 8 souches de R. mannitolilytica, isolées de culture de sang, étaient faibles productrices de biofilm/non-adhérentes.

Ensuite, l'aptitude à former un biofilm a été étudiée pour différentes bactéries à Gram positif. Plus précisément, des souches de Staphylococcus aureus, qui ont été associées à différents types d'infections humaines, comprenant des infections de la peau, endocardite, infections osseuses, choc septique et infections chroniques associées à un biofilm (Otto, 2008) ont été évaluées. Les souches de référence Sa6538 et Sa25923, connues pour former des biofilms (Latimer, 2012; Croes, 2009), ont été confirmées en tant que productrices forte et intermédiaire de biofilm (figure 2A et tableau 4a). Parmi les isolats cliniques, cinq souches ont été jugées productrices intermédiaires de biofilm (Sa3074-IFO, Sa3050-IFO, Sa0186-IFO, Sa5674-IFO et Sa5826-IFO) et trois souches, isolées de patients souffrant d'ulcères chroniques, ont été classées comme fortes productrices de biofilm (Sa3146-IFO, Sa3079-IFO et Sa3065-IFO). Parmi les quatre souches MRSA, deux étaient intermédiaires (Sa0186-IFO, Sa5826-IFO) et deux fortes productrices de biofilm, (Sa3146-IFO, et Sa3065-IFO). Les deux seules souches faibles productrices de biofilm provenaient d'enfants atteints de dermatite atopique légère (Sa3032-IFO et Sa0073-IFO). Aucune des souches analysées n'a été classée comme non-adhérente ou non productrice. Ce résultat est en accord avec les précédents rapports décrivant l'efficacité de S. aureus à produire du biofilm (Otto, 2008; Periasamy, 2012).

Staphylococcus epidermidis, composant majeur de la population microbienne normale de la peau humaine, est un pathogène nosocomial important chez les patients présentant des facteurs de prédisposition tel qu'une sonde ou un dispositif implanté (Otto, 2009). Bien que, S. epidermidis possède un potentiel pathogène faible, il a été isolé dans 40% des bactériémies ou infection du sang (Suetens, 2007). Cette forte occurrence provient probablement du fait qu'il est un colonisateur ubiquitaire de la peau humaine, et par conséquent une possible source de contamination de dispositifs lors de leur insertion / retrait (Otto, 2009). Néanmoins, la capacité de S. epidermidis à former un biofilm est considérée comme le déterminant le plus pertinent de sa virulence (O'Gara, 2001; Götz, 2002; Cerca, 2005). Dans nos tests les souches de référence Se12228 (Zhang, 2003) et Se14990 (Stepanovic, 2003) ont été classées respectivement en tant que producteurs de biofilm faibles et intermédiaires (figure 2B et le tableau 4B). Ces résultats sont en accord avec les données disponibles dans la littérature. L'analyse de la capacité d'adhérence par le test a révélé que 5 des 6 isolats cliniques dérivés de cultures de sang (Se5287-IFO, Se5669-IFO, Se5934-IFO, Se5752-IFO et Se5845-IFO), étaient des producteurs faibles de biofilm. Une souche isolée d'infection sanguine associée à un cathéter (Se5993-IFO) s'est révélée être une productrice de biofilm intermédiaire. Les deux autres souches de S. epidermidis, isolées de plaies ont toutes deux été classées en tant que productrices intermédiaires de biofilm.

**A)**

| McF | Sa6538 | Sa25923 | Sa3074-IFO | Sa3032-IFO | Sa3050-IFO | Sa0073-IFO | Sa0186-IFO | Sa5674-IFO | Sa5826-IFO | Sa3146-IFO | Sa3079-IFO | Sa3065-IFO | |
|---|---|---|---|---|---|---|---|---|---|---|---|---|---|
| $1\times10^{-1}$ | 1.00 | 1.00 | 1.00 | 1.00 | 1.00 | 1.00 | 1.00 | 1.00 | 1.00 | 1.00 | 1.00 | 1.00 | Non-adhérent/Faible |
| $1\times10^{-2}$ | 0.99 | 0.99 | 0.98 | 0.99 | 0.94 | 1.00 | 1.00 | 0.99 | 0.99 | 0.98 | 0.99 | 0.99 | Faible |
| $1\times10^{-3}$ | 0.95 | 0.99 | 0.92 | 0.09 | 0.84 | 0.44 | 0.98 | 0.99 | 0.98 | 0.98 | 0.99 | 0.97 | Intermédiaire |
| $1\times10^{-4}$ | 0.05 | 0.94 | 0.11 | 0.10 | 0.08 | 0.04 | 0.56 | 0.45 | 0.33 | 0.98 | 0.97 | 0.98 | Intermédiaire |
| $1\times10^{-5}$ | 0.09 | 0.54 | 0.12 | 0.03 | 0.10 | 0.04 | 0.05 | 0.02 | 0.02 | 0.93 | 0.74 | 0.99 | Fort |
| $1\times10^{-6}$ | 0.03 | 0.01 | 0.03 | 0.04 | 0.05 | 0.03 | 0.00 | 0.03 | -0.01 | 0.70 | 0.64 | 0.80 | Fort |
| | 1 | 2 | 3 | 4 | 5 | 6 | 7 | 8 | 9 | 10 | 11 | 12 | |

**B)**

| McF | Se12228 | Se14990 | Se5287-IFO | Se5669-IFO | Se5899-IFO | Se1501-IFO | Se5934-IFO | Se5752-IFO | Se5845-IFO | Se5993-IFO | |
|---|---|---|---|---|---|---|---|---|---|---|---|
| $1\times10^{-1}$ | 0.99 | 1.00 | 0.94 | 0.97 | 0.99 | 0.98 | 0.97 | 0.99 | 0.99 | 1.00 | Non-adhérent/Faible |
| $1\times10^{-2}$ | 0.59 | 0.99 | 0.62 | 0.98 | 0.99 | 0.91 | 0.84 | 0.99 | 0.99 | 0.98 | Faible |
| $1\times10^{-3}$ | 0.29 | 0.81 | 0.27 | 0.28 | 0.64 | 0.53 | 0.45 | 0.46 | 0.34 | 0.98 | Intermédiaire |
| $1\times10^{-4}$ | 0.20 | 0.27 | 0.20 | 0.00 | 0.05 | 0.20 | 0.16 | 0.12 | 0.05 | 0.87 | Intermédiaire |
| $1\times10^{-5}$ | 0.13 | 0.28 | 0.13 | 0.01 | 0.03 | 0.02 | 0.03 | 0.01 | 0.02 | 0.44 | Fort |
| $1\times10^{-6}$ | 0.01 | 0.02 | 0.03 | 0.06 | 0.02 | 0.02 | 0.05 | 0.03 | 0.05 | 0.01 | Fort |
| | 1 | 2 | 3 | 4 | 5 | 6 | 7 | 8 | 9 | 10 | |

**C)**

| McF | Sa6538 | Ss5800-IFO | Sh5529-IFO | Sh5592-IFO | Sag0140-IFO | Sag0093-IFO | Efm5304-IFO | Efm5515-IFO | Efs0044-IFO | Efs5269-IFO | |
|---|---|---|---|---|---|---|---|---|---|---|---|
| $1\times10^{-1}$ | 1.00 | 0.87 | 0.96 | 1.00 | 0.93 | 1.00 | 0.62 | 0.94 | 0.98 | 0.07 | Non-adhérent/Faible |
| $1\times10^{-2}$ | 0.99 | 0.21 | 0.83 | 0.90 | 0.97 | 1.00 | 0.48 | 0.83 | 0.97 | 0.06 | Faible |
| $1\times10^{-3}$ | 0.94 | 0.13 | 0.35 | 0.64 | 0.91 | 0.95 | 0.12 | 0.62 | 0.95 | 0.07 | Intermédiaire |

| | 1 | 2 | 3 | 4 | 5 | 6 | 7 | 8 | 9 | 10 | |
|---|---|---|---|---|---|---|---|---|---|---|---|
| 1x10⁻⁴ | 0.06 | -0.06 | 0.07 | 0.12 | 0.87 | 0.81 | -0.02 | 0.15 | 0.39 | 0.05 | Intermédiaire |
| 1x10⁻⁵ | 0.04 | 0.14 | 0.06 | 0.01 | 0.22 | 0.63 | -0.03 | 0.05 | -0.04 | 0.05 | Fort |
| 1x10⁻⁶ | 0.01 | 0.04 | 0.04 | 0.03 | 0.24 | 0.52 | 0.00 | 0.06 | 0.03 | 0.06 | Fort |

Tableau 4 : analyses de plaques obtenues avec le procédé A) S. aureus B) S. epidermidis et C) Divers bactéries à Gram positif.

D'autres bactéries à Gram positif, reconnues comme agents pathogènes nosocomiaux importants, ont été analysées. La souche de S. aureus Sa6538 a été utilisée comme souche de référence et les bactéries analysées incluaient: Staphylococcus haemolyticus, Streptococcus sanguinis, Streptococcus agalactiae, Enterococcus faecium et Enterococcus faecalis. Plus précisément, les souches de S. agalactiae (Sag0140-IFO et Sag0093-IFO) isolées d'ulcères infectés, ont été trouvées être des productrices intermédiaires / fortes de biofilm (figure 2C et le tableau 4C). E. faecium (Efm5304-IFO) a été classée comme faible productrice de biofilm alors que la souche d'ERV (Efm5515-IFO) a été identifiée comme productrice intermédiaire (figure 3C et 4C).

Analyse de la distribution espèce-spécifique du phénotype production de biofilm

L'analyse de la production de biofilm en fonction des différentes espèces bactériennes indique que parmi les bactéries à Gram négatif, P. aeruginosa avait le phénotype producteur de biofilm le plus cohérent. En fait, 6 souches (67%) étaient des fortes productrices de biofilm, 2 (22%) étaient des productrices intermédiaires et 1 (11%) était une faible productrice de biofilm, respectivement (figure 3). Inversement, 4 (50%) des isolats K. pneumoniae étaient producteurs intermédiaires de biofilm et 4 souches (50%) se sont révélés être des bactéries non-adhérentes/non productrices. Fait intéressant, les deux isolats cliniques et les souches de laboratoire de R. mannitolilytica, avaient la capacité d'adhérence plus faible. Parmi les bactéries à Gram positif, 3 (20%) des souches de S. aureus étaient des fortes productrices de biofilm, 5 (50%) étaient productrices intermédiaires et 2 ont été jugées faibles productrices de biofilm (20%). Dans le groupe d'isolats cliniques S. epidermidis, 6 souches avaient un phénotype faible productrice de biofilm (75%), tandis que les 2 autres (25%) souches étaient productrices intermédiaires de biofilm. Globalement, l'analyse des 52 isolats cliniques a révélé que plus de 44% (23/52) étaient productrices fortes/intermédiaires de biofilm et 85% (44/52) étaient capables de produire un biofilm. Malgré le fait que la répartition de ces 52 isolats ne reflète pas la réalité clinique, cette valeur est en corrélation avec les 80% estimés par Romling et Balsalobre (Romling, 2012; NIH Parent Grant Announcement, 2002). Les résultats les plus pertinents ont été obtenus avec P. aeruginosa (89%) et S. aureus (80%) en tant que productrices de biofilm les plus efficaces (figure 3).

Il est intéressant de noter que les souches avec de multiples résistances médicamenteuses se sont toutes révélées appartenir aux groupes productrices fortes/intermédiaires de biofilm. En effet, comme indiqué précédemment, les organismes multirésistants sont plus fréquemment associés à une forte production de biofilms (Kwon, 2008; Rao, 2008; Sanchez, 2013). Ce point semble représenter un élément clé qui peut favoriser la résistance antimicrobienne en sélectionnant les souches hautement résistantes exposées à des concentrations antimicrobienne sous-inhibitrices et en offrant des conditions favorables pour le transfert de gènes (Wang, 2010).

Catégorisation avec la coloration de cristal violet (CV).

Le phénotype de production de biofilm des isolats cliniques a en outre été évalué par le dosage au CV. L'absorption moyenne de la lumière pour les différentes bactéries produisant un biofilm obtenus par analyse au CV est représenté à la figure 6. Les résultats ont révélé que la reproductibilité de l'essai CV était généralement bonne avec des différences mineures observées entre les moyennes des résultats des répétitions. Dans les isolats cliniques et les souches de référence la formation de biofilm était hétérogène selon les espèces bactériennes et les isolats d'une même espèce.

Les résultats de l'essai au CV ont ensuite été comparés avec les données recueillies par le procédé selon l'invention. La concordance complète a été définie comme le pourcentage d'isolats qui étaient dans la même catégorie avec les deux méthodes; la concordance a été considérée comme partielle lorsque le procédé selon l'invention a obtenu le même classement que le CV DO570 $\pm$ l'écart type. Les isolats non cohérents ont été considérés comme étant en désaccord.

La concordance complète et partielle entre le procédé de caractérisation et le dosage CV pour P. aeruginosa était de 83% et 92%, correspondant à deux et un non cohérents respectivement. Pour K. pneumoniae le procédé de caractérisation a montré une précision partielle de 70% par rapport à la coloration CV, avec trois non cohérents. Dans le détail, deux souches classées comme non-adhérentes par le procédé de caractérisation (Kp3040-IFO; Kp5783-IFO) ont été classées comme faibles productrices de biofilm avec le CV et une souche intermédiaire (Kp0068-IFO) avec le procédé de caractérisation était forte productrice de biofilm selon la coloration CV. Une explication possible de cette concordance réduite pour K. pneumoniae pourrait être liée à la production de mucus épais dans ces souches qui pourraient avoir partiellement influencé la cohérence entre les tests. En effet, il a été démontré que les tests de CV peuvent être parfois faussés en raison des propriétés de coloration non spécifiques (Pan, 2010; Merritt, 2005; Skogman, 2012). Les souches de R. mannitolilytica ont montré une cohérence entre les tests de 90%, correspondant à une erreur de classification. Une cohérence complète entre les tests a été observée à la fois pour S. aureus et S. epidermidis, alors que pour d'autres bactéries à Gram positif la cohérence était de 87% correspondant à une erreur de classification (Ef5515-IFO). Globalement, la cohérence complète pour les souches à Gram négatif était de 68% alors que pour les bactéries à Gram positif, il était de plus de 77% montrant un accord global de 72,5% des échantillons analysés. Ainsi la cohérence partielle était de plus de 95% correspondant à 3 erreurs de classification dans 63 souches analysées. Il est important de noter que dans tous les résultats discordants, le classement diffère par une seule catégorie.

Ainsi, la cohérence totale a été analysée pour le procédé de classification et la coloration CV, en utilisant le test de McNemar. Les résultats indiquent que, pour les identifications discordantes, une différence statistiquement significative existe entre les tests (p = 0,007). Plus précisément, le procédé de classification sous-estime la production de biofilm par rapport au CV. Ce résultat n'a rien de surprenant car il résulte de la propriété de coloration non-spécifique du CV. En effet, le cristal violet est connu pour se lier à des molécules de surfaces chargées négativement, qui sont présentes à la fois sur les bactéries et la matrice extracellulaire du biofilm (Extremina, 2011). Cela peut conduire à une surestimation de la capacité réelle d'adhésion de différentes souches (Pan, 2010; Merritt, 2005; Skogman, 2012).

Tableau 5 : résultats globaux d'adhésion des différentes espèces bactériennes obtenues par le procédé de caractérisation et la coloration CV

| Espèces bactériennes | Procédé de caractérisation | | | | Coloration CV | | | | Cohérence (%) | |
|---|---|---|---|---|---|---|---|---|---|---|
| | Na | W | M | H | Na | W | M | H | Totale | Totale et Partielle |
| *P. aeruginosa* | 0 | 2 | 3 | 7 | 0 | 2 | 3 | 7 | 83 | 100 |
| *K. pneumoniae* | 5 | 0 | 5 | 0 | 1 | 4 | 3 | 2 | 40 | 80 |
| *R. mannitolilytica* | 1 | 9 | 0 | 0 | 0 | 10 | 0 | 0 | 80 | 100 |
| S. *aureus* | 0 | 2 | 6 | 4 | 0 | 1 | 7 | 4 | 92 | 100 |
| S. *epidermidis* | 0 | 7 | 3 | 0 | 0 | 7 | 2 | 1 | 70 | 100 |
| Autres Gram + | 1 | 3 | 4 | 1 | 0 | 6 | 1 | 2 | 60 | 90 |
| Na : non producteur, W : faible producteur, M : producteur intermédiaire, H : fort producteur de biofilm | | | | | | | | | | |

La concordance entre le procédé de caractérisation selon l'invention et le CV a été statistiquement mesurée par le coefficient kappa. Les résultats ont montré un bon accord entre les tests pour les producteurs de biofilm faibles (kappa = 0,71 $\pm$ 0,09; spécificité = 94,3%; précision = 85,7%), intermédiaires (kappa = 0,63 $\pm$ 0,11; spécificité = 84,8%; précision = 84,1%) et forts ( kappa = 0,73 $\pm$ 0,10; spécificité = 97,9%; précision = 90,5%), tandis que pour les cellules non-adhérentes / non productrice la cohérence était modérée (kappa = 0,42 $\pm$ 0,2; spécificité = 91,8%; précision = 92,1%). Dans l'ensemble, la force de cohérence entre ces méthodes était bonne (kappa = 0,66 $\pm$ 0,07), en particulier le procédé de caractérisation démontrant des niveaux élevés de spécificité et de précision (spécificité = 92,2%; précision = 88,1%).

Cet exemple démontre donc clairement que le procédé de caractérisation, également désigné cBFRT, est un procédé rapide et fiable pour une évaluation quantitative de la production de biofilm bactérien. Ce procédé a été testé avec différentes espèces bactériennes, avec différents phénotypes de formation biofilm, incluant des souches de laboratoire et des isolats cliniques. La capacité du procédé à évaluer la production de biofilm a été comparée avec le test de CV, qui est une méthode largement utilisée pour la quantification biofilm (Christensen, 1985; Stepanovic, 2000, Peeters, 2008). D'après le coefficient de kappa, la conformité globale entre les tests était bonne (kappa = 0,66 $\pm$ 0,07) avec le procédé montrant une spécificité (92,3%) et une précision (88,1%) élevées pour classer les bactéries productrices de biofilm. Notamment, pour tous les résultats de souches de références le procédé étaient en accord/cohérent avec les données rapportées dans la littérature. La cohérence catégorielle complète entre le procédé et la coloration CV était de 72,5% tandis que la cohérence catégorielle partielle a dépassé 96%, avec seulement deux échantillons mal classés dans les 63 souches analysées. La cohérence catégorielle complète était de 68% pour les à Gram négatif et 77% pour les bactéries à Gram positif alors que la cohérence catégorielle partielle était respectivement de 93,3%, et 96,6. Le nombre plus élevé de résultats discordants se situe dans le groupe des faibles producteurs de biofilm / non-adhérents et, en particulier, toutes ces souches ont montré un phénotype mucoïde qui a affecté la sensibilité du test de CV. Il convient de noter que le CV est un dosage colorimétrique non spécifique, qui colore les bactéries vivantes et mortes, ainsi que la matrice du biofilm. Ainsi, ce test, qui prévoit seulement une quantification indirecte du biofilm, peut conduire à une surestimation des résultats (Pan, 2010; Merritt, 2005; Skogman, 2012), en particulier lors de l'essai sur souches mucoïdes. En effet, il a été démontré que la surproduction de mucus ne joue pas un rôle significatif dans l'adhésion bactérienne et la formation de matrice biofilm, mais il a un rôle protecteur contre la réponse immunitaire de l'hôte (Stapper, 2004; Leid, 2005). A l'inverse, le procédé de classification fournit une estimation directe de la véritable capacité de différentes souches bactériennes d'agréger ou adhérer, assurant ainsi une plus grande spécificité. En fait, la spécificité différente des deux méthodes a été indirectement confirmée par le test de McNemar, qui a révélé que, dans les cas où les méthodes sont en désaccord, le procédé de classification a une tendance significative (P = 0,007) à sous-estimer les résultats. Les autres différences importantes entre le procédé de classification et le CV sont liées à la préparation et le temps d'exécution ainsi que la reproductibilité des données. Avec le procédé de classification, la préparation d'une pleine plaque et l'inoculation nécessite, par exemple environ 30 minutes plus par exemple cinq heures d'incubation et par exemple quelques minutes pour l'analyse de la plaque. A l'inverse, le dosage CV a permis une détection de biofilm seulement après 24/48 heures d'incubation et nécessite des étapes de lavages répétées et de laborieuses procédures de coloration (Stepanovic, 2000; Djordjevic, 2002). La nécessité de plusieurs manipulations non normalisées dans le test de CV a été associée à de grandes variations intra- et inter-expériences conduisant à des écarts-types importants (Peeters, 2008). Le manque de reproductibilité est également l'un des principaux inconvénients posés par d'autres techniques classiques en particulier dans le criblage à haut débit (Peeters, 2008).

Néanmoins, le développement de méthodes normalisées pour identifier le phénotype de bactéries est d'une importance capitale dans la pratique clinique, car il peut ajouter un élément de prise de décision clé dans le soutien à une gestion thérapeutique efficace des «infections difficiles", telles que celles associés à des dispositifs chirurgicaux (à savoir les infections associées aux cathéters), qui aboutissent souvent à l'échec du traitement et l'élimination de l'appareil, malgré la mise en œuvre de stratégies thérapeutiques apparemment appropriées (Costerton, 2003). En fait, une fois que le biofilm est établi sur le dispositif, les cellules individuelles présentent une tolérance accrue à des agents antimicrobiens et le traitement antibiotique seul est souvent insuffisant. Des expériences in vitro et in vivo ont démontré que dans une matrice de biofilm biologique, les cellules montrent une concentration minimale inhibitrice beaucoup plus élevée (CMI) (environ 10-1000 fois) que les mêmes cellules bactériennes examinées dans des conditions de croissance planctoniques (Høiby, 2011 ; Hengzhuang, 2012). La CMI antibiotique efficace in vivo pour l'éradication de biofilm peut donc être impossibles à atteindre par l'administration d'antibiotiques à des doses qui semblent efficaces pour la croissance planctonique, en raison de la toxicité et des effets secondaires des médicaments, y compris les limitations imposées par les fonctions rénale et / ou hépatiques. Ainsi, la reconnaissance en temps opportun d'un fort producteur de biofilm, avant le développement d'une matrice de biofilm mature, peut contribuer au ciblage approprié de l'intervention thérapeutique (type, doses, durée) et de prise de décision (par exemple le retrait du cathéter).

Tel que démontré, le procédé de classification a été développé pour fonctionner en combinaison avec les procédures cliniques traditionnelles de microbiologie, sans altérer la routine quotidienne de travail. Immédiatement après l'isolement du micro-organisme, par exemple sur une plaque de gélose, il est possible d'analyser la capacité de production de biofilm.

Tel que démontré dans cet exemple, le procédé de l'invention permet de caractériser des microorganismes producteurs de biofilm, par exemple des bactéries, des levures etc. Il est donc évident que le procédé peut être utilisé pour la caractérisation de formation de biofilms par d'autres organismes tels que levures, avec un intérêt et des applications similaires à celles revendiquées pour les bactéries.

En outre le procédé de classification peut fournir des informations sur la capacité d'un isolat bactérien inconnu à former un biofilm avant même l'identification du micro-organisme avec les techniques phénotypiques classiques. Ainsi, le procédé selon l'invention trouve de nombreuses applications, par exemple dans le domaine dentaire où les biofilms sont associés avec les maladies dentaires majeures telles que la carie dentaire et les maladies parodontales. En plus de ces applications cliniques, le procédé de classification peut représenter un outil précieux par exemple pour l'industrie agro-alimentaire ainsi que l'industrie de l'assainissement, y compris les systèmes d'eau.

## Listes des références

1. Benoit MR, Conant CG, Ionescu-Zanetti C, Schwartz M, Matin A. New Device for High-Throughput Viability Screening of Flow Biofilms. Appl Env ss, 2010, 76 (13), 4136-4142
2. Biofiles issue 3.8, 21-24
3. Branger A. Microbiochimie et alimentation, Educagri Editions, 2012
4. Cerca N, Martins S, Cerca F, Jefferson KK, Pier GB, Oliveira R, Azeredo J. Comparative assessment of antibiotic susceptibility of coagulase-negative staphylococci in biofilm versus planktonic culture as assessed by bacterial enumeration or rapid XTT colorimetry. J Antimicrob Chemother. 2005; 56:331-6.
5. Chavant P, Gaillard-Martinie B, Talon R, Hébraud M, Bernardi T A new device for rapid evaluation of biofilm formation potential by bacteria.. J Microbiol Methods. 2007;68(3):605-12
6. Christensen, G.D., Simpson, W.A., Younger, J.J., Baddour, L.M., Barrett, F.F., Melton, D.M., Beachey, E.H., 1985. Adherence of coagulase-negative staphylococci to plastic tissue culture plates: a quantitative model for the adherence of staphylococci to medical devices. J. Clin. Microbiol. 22,996-1006.
7. Coenye T, Spilker T, Reik R, Vandamme P, Lipuma JJ. Use of PCR analyses to define the distribution of Ralstonia species recovered frompatientswith cystic fibrosis. J Clin Microbiol 2005, 43:3463-3466.
8. Costerton, J. W., P. S. Stewart, and E. P. Greenberg. 1999. Bacterial biofilms: a common cause of persistent infections. Science 284:1318-1322.
9. Costerton W, Veeh R, Shirtliff M, Pasmore M, Post C, Ehrlich G. The application of biofilm science to the study and control of chronic bacterial infections. J Clin Invest. 2003;112:1466-1477.
10. Croes S, Deurenberg RH, Boumans ML, Beisser PS, Neef C, Stobberingh EE. Staphylococcus aureus biofilm formation at the physiologic glucose concentration depends on the S. aureus lineage. BMC Microbiol. 2009; 28; 9:229.

11. Darouiche RO. Treatment of infections associated with surgical implants. N Engl J Med 2004; 350: 1422-1429

12. De Baere T, Steyaert S, Wauters G, et al. Classification of Ralstonia pickettii biovar 3/'thomasii' strains (Pickett 1994) and of new isolates related to nosocomial recurrent meningitis as Ralstonia mannitolytica sp. nov. Int J Syst Evol Microbiol. 2001; 51: 547-58.

13. De Gheldre Y, Avesani V, Berhin C, Delmée M, Glupczynski Y. Evaluation of Oxoid combination discs for detection of extended-spectrum beta-lactamases. J Antimicrob Chemother. 2003 Oct;52(4):591-7.

14. Dissemond J. When is a wound chronic? Hautarzt 2006; 57: 55.

15. Djordjevic D, Wiedmann M, McLandsborough LA. Microtiter Plate Assay for Assessment of Listeria monocytogenes Biofilm Formation. Appl Env Microbiol, 2002, 68 (6), 2950-2958

16. Donlan RM. Biofilms and device-associated infections. Emerg Infect Dis 2001; 7(2): 277-81.

17. Eng RH, Smith SM, Cherubin C. Inoculum effect of new beta-lactam antibiotics on Pseudomonas aeruginosa. Antimicrob Agents Chemother. 1984; 26(1):42-7

18. Extremina CI, Costa L, Aguiar AI, Peixe L, Fonseca AP. Optimization of processing conditions for the quantification of enterococci biofilms using microtitre-plates. J Microbiol Meth 2011, 84, 167-173

19. Furuya EY, Lowy FD: Antimicrobial strategies for the prevention and treatment of cardiovascular infections. Curr Opin Pharmacol 2003,3(5):464-469.

20. Gbejuade HO, Lovering AM, Webb JC. The role of microbial biofilms in prosthetic joint infections - A review. Acta Orthopaedica 2015; 86 (2): 147-158

21. Gotz F. Staphylococcus and biofilms. Mol Microbiol 2002; 43: 1367-1378.

22. Hengzhuang W, Wu H, Ciofu O et al. In vivo pharmacokinetics/pharmacodynamics of colistin and imipenem in Pseudomonas aeruginosa biofilm infection. Antimicrob Agents Chemother 2012; 56(5): 2683-2690.

23. Høiby N., Ciofu O., Bjarnsholt T. Pseudomonas aeruginosa biofilms in cystic fibrosis, Future Microbiology. 2010a, 5;1663-1674.

24. Høiby N, Bjarnsholt T, Givskov M et al. Antibiotic resistance of bacterial biofilms. Int J Antimicrob Agents 2010b; 35(4): 322-332

25. Høiby N, CiofuO, JohansenHK et al. The clinical impact of bacterial biofilms. Int J Oral Sci 2011; 3(2): 55-65

26. Jacqueline C, Caillon J. Impact of Bacterial Biofilm On The Treatment of Prosthetic Joint Infections. J Antimicrob Chemother 2014;69:i37-i40

27. Joseph LA, Wright AC. Expression of Vibrio vulnificus Capsular Polysaccharide Inhibits Biofilm Formation. J Bacteriol, 2004, 186 (3) 889-893

28. Kurtz SM, Lau E, Watson H, Schmier JK, Parvizi J. Economic Burden of Periprosthetic Joint Infection in the United States. J Arthroplasty, 2012, 27(8) 61-65

29. Kwon AS, Park GC, Ryu SY, Lim DH, Lim DY, Choi CH, Park Y, Lim Y: Higher biofilm formation in multidrug-resistant clinical isolates of Staphylococcus aureus. Int J Antimicrob Agents 2008, 32:68-72.

30. Latimer J, Forbes S, McBain AJ. Attenuated virulence and biofilm formation in Staphylococcus aureus following sublethal exposure to triclosan. Antimicrob Agents Chemother. 2012 Jun;56(6):3092-100.

31. Leid JG, Willson CJ, Shirtliff ME, Hassett DJ, Parsek MR, Jeffers AK. The exopolysaccharide alginate protects Pseudomonas aeruginosa biofilm bacteria from IFN-gamma-mediated macrophage killing. J Immunol. 2005 Dec 1;175(11):7512-8.

32. Lindsay, D., von Holy, A. Bacterial biofilms within the clinical setting: what healthcare professionals should know. J Hosp Infect 2006; 64: 4, 313-325.

33. Marroni M, Pasticci MB, Pantosti A, Colozza MA, Stagni G, Tonato M. Outbreak of infusion-related septicemia by Ralstonia pickettii in the Oncology Department. Tumori. 2003; 89: 575-6.

34. Merritt JH, Kadouri DE, O'Toole GA. Growing and Analyzing Static Biofilms. Curr Protoc Microbiol. 2005 July ; 0 1: Unit-1B.1. doi:10.1002/9780471729259.mc01b01s00

35. Moremi N, Mushi MF, Fidelis M, Chalya P, Mirambo M, Mshana SE. Predominance of multi-resistant Gram-negative bacteria colonizing chronic lower limb ulcers (CLLUs) at Bugando Medical Center. BMC Res Notes. 2014 Apr 4;7:211.

36. Müsken M, Di Fiore S, Römling U, Häussler S. A 96-well-plate-based optical method for the quantitative and qualitative evaluation of Pseudomonas aeruginosa biofilm formation and its application to susceptibility testing. Nature Protocols (2010), 5 (8) 1460-1469

37. Naparstek L, Carmeli Y, Navon-Venezia S, Banin E. Biofilm formation and susceptibility to gentamicin and colistin of extremely drug-resistant KPC-producing Klebsiella pneumoniae. J Antimicrob Chemother. 2014;;69(4):1027-34.

38. NIH Parent Grant Announcement, 2002. Research on Microbial Biofilms. NIH website. Available: http://grants.nih.gov/grants/guide/pafiles/PA-03-047.html. Accessed 2014 Sept 2.

39. O'Gara JP & Humphreys H.Staphylococcus epidermidis biofilms: importance and implications. J Med Microbiol. 2001; 50:582-587.

40. Olivares E, Badel-Berchoux S, Provot C, Jaulhac B, Prévost G, Bernardi T, Jehl F. The Biofilm Ring Test®: a rapid method for the routine analysis of P. aeruginosa biofilm formation kinetics. JCM Accepted Manuscript Posted Online 30 December 2015. J. Clin. Microbiol. doi:10.1128/JCM.02938-15

41. Otto M (2008) Staphylococcal biofilms. Curr Top Microbiol Immunol 322:207-228.

42. Otto M. Staphylococcus epidermidis - the 'accidental' pathogen.Nat Rev Microbiol. 2009 Aug;7(8):555-67.

43. Pan Y, Breidt F, Gorski L. Synergistic Effects of Sodium Chloride, Glucose, and Temperature on Biofilm Formation by Listeria monocytogenes Serotype 1/2a and 4b Strains. Appl Env Microbiol, 2010, 76 (5), 1433-1441

44. Peeters E, Nelis HJ, Coenye T. Comparison of multiple methods for quantification of microbial biofilms grown in microtiter plates. J Microbiol Methods. 2008 Feb;72(2):157-65.

45. Periasamy S, Joo HS, Duong AC, Bach TH, Tan VY, Chatterjee SS, Cheung GY, Otto M. How Staphylococcus aureus biofilms develop their characteristic structure. Proc Natl Acad Sci U S A. 2012; 24;109(4):1281-6.

46. Podschun R, and Ullmann U. Klebsiella spp. as nosocomial pathogens: epidemiology, taxonomy, typing methods and pathogenicity factors. Clin Microbiol Rev 1998, 11:589-603.

47. Rahme LG, Stevens EJ, Wolfort SF, Shao J, Tompkins RG, Ausubel FM. Common virulence factors for bacterial pathogenicity in plants and animals. Science. 1995;268:1899-902.

48. Rao RS, Karthika RU, Singh SP, Shashikala P, Kanungo R, Jayachandran S, Prashanth K: Correlation between biofilm production and multiple drug resistance in imipenem resistant clinical isolates of Acinetobacter baumannii. Indian J Med Microbiol 2008, 26:333-337.

49. Romling U, Balsalobre C: Biofilm infections, their resilience to therapy and innovative treatment strategies. J Intern Med 2012, 272(6):541-561.

50. Ryan MP, Adley CC. Ralstonia spp.: emerging global opportunistic pathogens. Eur J Clin Microbiol Infect Dis. 2014.

51. Sanchez CJ Jr, Mende K, Beckius ML, Akers KS, Romano DR, Wenke JC, Murray CK. Biofilm formation by clinical isolates and the implications in chronic infections. BMC Infect Dis. 2013; 29; 13:47.

52. Sangetha S, Zuraini Z, Suryani S, Sasidharan S. In situ TEM and SEM studies on the antimicrobial activity and prevention of Candida albicans biofilm by Cassia spectabilis extract. Micron (2009) 40, 439-443

53. Schaber JA, Carty NL, McDonald NA, Graham ED, Cheluvappa R, Griswold JA, Hamood AN. Analysis of quorum sensing-deficient clinical isolates of Pseudomonas aeruginosa. J Med Microbiol. 2004;53:841-53.

54. Singh, P.K.; Schaefer, A.L.; Parsek, M.R.; Moninger, T.O.; Welsh, M.J.; Greenberg, E.P. Quorum-sensing signals indicate that cystic fibrosis lungs are infected with bacterial biofilms. Nature 2000, 407, 762-764.

55. Skogman ME, Vuorela PM, Fallarero A. Combining biofilm matrix measurements with biomass and viability assays in susceptibility assessments of antimicrobials against Staphylococcus aureus biofilms. J Antibiot (2012) 65, 453-459

56. Stapleton FDJ, Matheson M, Woodward E. Bacterial adherence and glycocalyx formation on unworn hydrogel lenses. J Brit Contact Lens Assoc. 1993;16:113-6.

57. Stapper AP, Narasimhan G, Ohman DE, Barakat J, Hentzer M, Molin S, Kharazmi A, Høiby N, Mathee K. Alginate production affects Pseudomonas aeruginosa biofilm development and architecture, but is not essential for biofilm formation. J Med Microbiol. 2004 Jul;53(Pt 7):679-90.

58. Stepanovic S, Vukovic D, Dakic I, Savic B, Svabic-Vlahovic M. A modified microtiter-plate test for quantification of staphylococcal biofilm formation. J Microbiol Methods. 2000;40:175-9.

59. Suetens C, Morales I, Savey A et al., "European surveillance of ICU-acquired infections (HELICS-ICU): methods and main results," Journal of Hospital Infection, vol. 65, no. 2, pp. 171-173, 2007.

60. Thermo Scientific Technical Bulletin B06a Principles in adsorption to polystyrene

61. Viera AJ, Garrett JM. Understanding interobserver agreement: the kappa statistic. Fam Med. 2005 May;37(5):360-3

62. Wang Q, Sun FJ, Liu Y, Xiong LR, Xie LL, Xia PY: Enhancement of biofilm formation by subinhibitory concentrations of macrolides in icaADBCpositive and -negative clinical isolates of Staphylococcus epidermidis. Antimicrob Agents Chemother 2010, 54:2707-2711.

63. Welch K, Cai Y, Strømme M. A Method for Quantitative Determination of Biofilm Viability. J. Funct. Biomater. 2012, 3, 418-431

64. Yang D and Zhang Z. Biofilm-forming Klebsiella pneumoniae strains have greater likelihood of producing extended-spectrum beta-lactamases. J. Hosp. Infect. 2008, 68, 369-371.

65. Zhang YQ, Ren SX, Li HL, Wang YX, Fu G, Yang J, Qin ZQ, Miao YG, Wang WY, Chen RS, Shen Y, Chen Z, Yuan ZH, Zhao GP, Qu D, Danchin A, Wen YM. Genome-based analysis of virulence genes in a non-biofilm-forming Staphylococcus epidermidis strain (ATCC 12228). Mol Microbiol. 2003 Sep;49(6):1577-93.

**Revendications**

1. Procédé de détermination de la capacité de production de biofilm par un microorganisme comprenant les étapes suivantes:

   a) Introduire dans des contenants de culture comprenant un milieu de culture liquide approprié au développement dudit microorganisme au moins deux particules, lesdites particules reposant sur une surface immergée dans le milieu de culture,
   b) Introduire et ensemencer indépendamment le milieu de culture des contenants obtenus à l'étape a) avec ledit microorganisme à une concentration comprise de $1 \times 10^{-1}$ à $1 \times 10^{-6}$ McFarland (McF), chaque milieu comprenant indépendamment une concentration différente de microorganismes,
   c) maintenir les milieux de culture ensemencés dans des conditions permettant un développement dudit microorganisme,
   d) appliquer un champ capable de mettre en mouvement lesdites au moins deux particules reposant sur une surface immergée dans le milieu de culture,
   e) détermination de la capacité de production de biofilm par le microorganisme par observation et mesure de l'agrégation desdites particules comme suit :

      - l'absence d'agrégation desdites particules apparaissant à une concentration comprise de $1 \times 10^{-6}$ à $1 \times 10^{-4}$ McF correspondant à un microorganisme fort producteur de biofilm,
      - l'absence d'agrégation desdites particules apparaissant à partir d'une concentration supérieure à $1 \times 10^{-4}$ et inférieure à $1 \times 10^{-2}$ McF correspondant à un microorganisme producteur intermédiaire de biofilm,
      - l'absence d'agrégation desdites particules apparaissant à partir d'une supérieure ou égale à $1 \times 10^{-2}$ McF correspondant à un microorganisme faible producteur de biofilm,
      - une agrégation desdites particules quelle que soit la concentration correspondant à un microorganisme non producteur de biofilm.

2. Procédé selon la revendication 1 dans lequel le procédé comprend une étape e') de détermination de la valeur de l'Index Biofilm (BFI) pour chacun des milieux par :

   - analyse des composantes des couleurs rouge, vert bleu des pixels de l'image permettant de déterminer la densité de particules par pixel,
   - calcul de la corrélation entre les particules,
   - calcul de la densité d'agrégation des particules exprimée en BFI, et

   détermination de l'indice de formation potentielle du biofilm par contenant (BPc) selon la formule (I) suivante :

   $$BPc= [1-(BFIe / BFIn)] \ (I)$$

   dans laquelle BFIe correspond à la valeur de l'index biofilm dans le milieu ensemencé et BFIn correspond à la valeur de l'index biofilm dans un contenant ne comprenant pas de microorganismes (contrôle négatif).

3. Procédé selon la revendication 2, comprenant une étape e") postérieure ou simultanée à l'étape e') de la détermination de la valeur seuil (S) selon la formule (II) suivante :

   $$S= 1 - [(mBFIn - 3 \ X \ stmBFIn)/2] / mBFIn \ (II)$$

   dans laquelle mBFIn est égale à la moyenne des BFI des contrôles négatifs, stmBFIn est également à la déviation standard de la moyenne des BFI des contrôles négatifs calculée.

4. Procédé selon la revendication 3, dans lequel la capacité de production de biofilm par le microorganisme est déterminée par comparaison de la valeur de BPc en fonction de la concentration de microorganismes comme suit :

   - une valeur de BPc supérieure ou égale à la valeur seuil S à partir d'une concentration de microorganismes comprise de $1 \times 10^{-6}$ à $1 \times 10^{-4}$ McF correspondant à un microorganisme fort producteur de biofilm,
   - une valeur de BPc supérieure ou égale à la valeur seuil S à partir d'une concentration de microorganismes

supérieure à $1\times10^{-4}$ et inférieure à $1\times10^{-2}$ McF correspondant à un microorganisme producteur intermédiaire de biofilm,

- une valeur de BPc supérieure ou égale à la valeur seuil S à partir d'une concentration de microorganismes de $1\times10^{-2}$ McF correspondant à un microorganisme faible producteur de biofilm,
- une valeur de BPc inférieure à la valeur seuil S quelque soit la concentration correspondant à un microorganisme non producteur de biofilm.

5. Procédé de classification de microorganismes comprenant les étapes suivantes:

a) Introduire dans des contenants de culture comprenant un milieu de culture liquide approprié au développement dudit microorganisme au moins deux particules, lesdites particules reposant sur une surface immergée dans le milieu de culture,

b) Introduire et ensemencer indépendamment le milieu de culture des contenants obtenus à l'étape a) avec ledit microorganisme à une concentration comprise de $1\times10^{-1}$ à $1\times10^{-6}$ McF, chaque milieu comprenant indépendamment une concentration différente de microorganismes,

c) maintenir les milieux de culture ensemencés dans des conditions permettant un développement dudit microorganisme,

d) appliquer un champ capable de mettre en mouvement lesdites au moins deux particules reposant sur une surface immergée dans le milieu de culture préférentiellement dans le but d'obtenir une agrégation, préférentiellement sous forme de spot, desdites au moins deux particules,

e) classer le microorganisme en fonction par observation de l'agrégation desdites particules comme suit :

- catégorie I : absence d'agrégation desdites particules apparaissant à une concentration comprise de $1\times10^{-6}$ à $1\times10^{-4}$ McF
- catégorie II : absence d'agrégation desdites particules apparaissant à partir d'une concentration supérieure à $1\times10^{-4}$ et inférieure à $1\times10^{-2}$ McF correspondant à un microorganisme producteur intermédiaire de biofilm,
- catégorie III: absence d'agrégation desdites particules apparaissant à partir d'une concentration supérieure ou égale à $1\times10^{-2}$ McF correspondant à un microorganisme faible producteur de biofilm,
- catégorie IV : agrégation desdites particules quelle que soit la concentration correspondant à un microorganisme non producteur de biofilm.

6. Procédé selon l'une quelconque des revendications précédentes, comprenant six contenants de culture dans lesquels la concentration de microorganismes à l'étape b) est respectivement de $1\times10^{-6}$ McF, $1\times10^{-5}$ McF, $1\times10^{-4}$ McF, $1\times10^{-3}$ McF, $1\times10^{-2}$ McF et $1\times10^{-1}$ McF.

7. Procédé selon l'une quelconque des revendications précédentes dans lequel le maintien des milieux de culture ensemencés dans des conditions permettant un développement dudit microorganisme est réalisé pendant 1 à 8 heures, de préférence 4 à 6 heures.

8. Procédé selon l'une quelconque des revendications 1 à 5 dans lequel le maintien des milieux de culture ensemencés dans des conditions permettant un développement dudit microorganisme est réalisé pendant 5 à 12 temps de génération dudit microorganisme.

9. Procédé selon l'une quelconque des revendications précédentes, dans lequel lesdites, au moins deux particules sont des particules chargées électriquement, magnétiques ou magnétisables ou recouvertes d'au moins une couche magnétique ou magnétisable.

10. Procédé selon l'une quelconque des revendications précédentes, dans lequel lesdites, au moins deux, particules sont soumises à un champ électromagnétique, éventuellement appliqué par impulsion.

11. Procédé selon l'une quelconque des revendications précédentes, dans lequel lesdites, au moins deux, particules sont soumises à une augmentation progressive du champ électromagnétique.

12. Procédé selon l'une quelconque des revendications précédentes, dans lequel ledit champ magnétique est généré par des moyens générateurs de champ en mouvement.

13. Procédé selon l'une quelconque des revendications précédentes, dans lequel lesdites, au moins deux, particules sont éclairées au moyen d'une source lumineuse pour détecter son mouvement.

14. Procédé selon l'une quelconque des revendications précédentes, dans lequel lesdites, au moins deux, particules sont génératrices d'un signal.

15. Procédé selon la revendication précédente, dans lequel lesdites, au moins deux, particules sont excitables, fluorescentes ou phosphorescentes ou radioactives ou chimioluminescentes.

16. Procédé selon l'une quelconque des revendications précédentes, dans lequel l'un desdits compartiments ne comprend pas ledit microorganisme, ledit compartiment représentant un témoin du milieu.

**Patentansprüche**

1. Verfahren zur Bestimmung der biofilmproduzierenden Kapazität eines Mikroorganismus, umfassend die folgenden Schritte:

a) Einbringen von mindestens zwei Partikeln in Kulturbehälter, die ein flüssiges, für das Wachstum des Mikroorganismus geeignetes Kulturmedium enthalten, wobei die Partikel auf einer in das Kulturmedium eingetauchten Oberfläche ruhen,
b) Einführen und unabhängiges Beimpfen des Kulturmediums der in Schritt a) erhaltenen Behälter mit dem Mikroorganismus in einer Konzentration von $1 \times 10^{-1}$ bis $1 \times 10^{-6}$ McFarland (McF), wobei jedes Medium unabhängig eine unterschiedliche Konzentration an Mikroorganismen enthält,
c) Halten der beimpften Kulturmedien unter Bedingungen, die das Wachstum des Mikroorganismus ermöglichen,
d) Anlegen eines Feldes, das in der Lage ist, die mindestens zwei Partikel zu bewegen, die auf einer in das Kulturmedium eingetauchten Oberfläche ruhen,
e) Bestimmung der Fähigkeit des Mikroorganismus zur Bildung eines Biofilms durch Beobachtung und Messung der Aggregation der Partikel wie folgt:

- das Fehlen der Aggregation der Partikel, die bei einer Konzentration von $1 \times 10^{-6}$ bis $1 \times 10^{-4}$ McF auftreten, was einem stark biofilmproduzierenden Mikroorganismus entspricht
- das Fehlen einer Aggregation der Partikel, die bei einer Konzentration von mehr als $1 \times 10^{-4}$ und weniger als $1 \times 10^{-2}$ McF auftreten, was einem mäßig biofilmproduzierenden Mikroorganismus entspricht,
- das Fehlen einer Aggregation der Partikel, die ab einer Konzentration von mindestens $1 \times 10^{-2}$ McF auftreten, was einem schwach biofilmproduzierenden Mikroorganismus entspricht,
- eine Aggregation der Partikel, unabhängig von der Konzentration, die einem nicht biofilmproduzierenden Mikroorganismus entspricht.

2. Verfahren nach Anspruch 1, wobei das Verfahren einen Schritt e') zur Bestimmung des Biofilm-Index (BFI)-Werts für jedes der Medien umfasst, indem:

- Analyse der roten, grünen und blauen Farbkomponenten der Pixel des Bildes, was die Bestimmung der Partikeldichte pro Pixel ermöglicht,
- die Berechnung der Korrelation zwischen den Partikeln,
- die Berechnung der Aggregationsdichte der Partikel, ausgedrückt als BFI, und
bestimmung des Biofilmbildungspotenzialindex pro Behälter (BPc) gemäß der folgenden Formel (I):

$$BPc= [1-(BFIe / BFIn)] \text{ (I)}$$

wobei BFIe dem Wert des Biofilmbildungsindexes in dem angeimpften Medium und BFIn dem Wert des Biofilmbildungsindexes in einem Behälter ohne Mikroorganismen (Negativkontrolle) entspricht.

3. Verfahren nach Anspruch 2, umfassend einen Schritt e") im Anschluss an oder gleichzeitig mit dem Schritt e') zur Bestimmung des Schwellenwertes (S) gemäß der folgenden Formel (II):

$$S= 1 - [(mBFIn - 3 \text{ X } stmBFIn)/2] / mBFIn \text{ (II)}$$

wobei mBFIn gleich dem Mittelwert der BFI der Negativkontrollen ist, stmBFIn gleich der berechneten Standardabweichung des Mittelwerts der BFI der Negativkontrollen ist.

4. Verfahren nach Anspruch 3, wobei die biofilmproduzierende Kapazität des Mikroorganismus durch Vergleich des BPc-Wertes in Abhängigkeit von der Konzentration der Mikroorganismen wie folgt bestimmt wird:

- ein BPc-Wert größer oder gleich dem Schwellenwert S ab einer Konzentration von Mikroorganismen von $1\times10^{-6}$ bis $1\times10^{-4}$ McF, was einem stark biofilmproduzierenden Mikroorganismus entspricht,
- ein BPc-Wert größer oder gleich dem Schwellenwert S bei einer Konzentration von Mikroorganismen von mehr als $1\times10^{-4}$ und weniger als $1\times10^{-2}$ McF, was einem mäßig biofilmproduzierenden Mikroorganismus entspricht,
- ein BPc-Wert, der größer oder gleich dem Schwellenwert S einer Konzentration von Mikroorganismen von $1\times10^{-2}$ McF ist, was einem schwach biofilmproduzierenden Mikroorganismus entspricht,
- einen BPc-Wert, der unabhängig von der Konzentration kleiner als der Schwellenwert S ist und einem nicht biofilmproduzierenden Mikroorganismus entspricht.

5. Verfahren zur Klassifizierung von Mikroorganismen, umfassend die folgenden Schritte:

a) Einbringen von mindestens zwei Partikeln in Kulturbehälter, die ein flüssiges, für das Wachstum des Mikroorganismus geeignetes Kulturmedium enthalten, wobei die Partikel auf einer in das Kulturmedium eingetauchten Oberfläche ruhen,
b) Einführen und unabhängiges Beimpfen des Kulturmediums der in Schritt a) erhaltenen Behälter mit dem Mikroorganismus in einer Konzentration von $1\times10^{-1}$ bis $1\times10^{-6}$ McF, wobei jedes Medium unabhängig voneinander eine unterschiedliche Konzentration an Mikroorganismen enthält,
c) Halten der beimpften Kulturmedien unter Bedingungen, die das Wachstum des Mikroorganismus ermöglichen,
d) Anlegen eines Feldes, das in der Lage ist, die mindestens zwei Teilchen, die auf einer in das Kulturmedium eingetauchten Oberfläche ruhen, zu bewegen, vorzugsweise mit dem Ziel, eine Aggregation, vorzugsweise in Form von Punkten, der mindestens zwei Teilchen zu erhalten,
e) Klassifizierung des Mikroorganismus durch Beobachtung der Aggregation der Partikel wie folgt:

- Kategorie I: Abwesenheit der Aggregation der Partikel, die in einer Konzentration von $1\times10^{-6}$ bis $1\times10^{-4}$ McF auftreten
- Kategorie II: Abwesenheit von Aggregation der Partikel, die bei einer Konzentration von mehr als $1\times10^{-4}$ und weniger als $1\times10^{-2}$ McF, was einem mäßig biofilmproduzierenden Mikroorganismus entspricht,
- Kategorie III: Abwesenheit von Aggregation der Partikel, die ab einer Konzentration von mindestens 1x10-2 McF auftreten, was einem schwach biofilmproduzierenden Mikroorganismus entspricht,
- Kategorie IV: Aggregation der genannten Partikel, unabhängig von der Konzentration, entsprechend einem nicht biofilmproduzierenden Mikroorganismus.

6. Verfahren nach einem der vorhergehenden Ansprüche, umfassend sechs Kulturbehälter, in denen die Konzentration der Mikroorganismen in Schritt b) jeweils $1\times10^{-6}$ McF, $1\times10^{-5}$ McF, $1\times10^{-4}$ McF, $1\times10^{-3}$ McF, $1\times10^{-2}$ McF und $1\times10^{-1}$ McF beträgt.

7. Verfahren nach einem der vorhergehenden Ansprüche, wobei die beimpften Kulturmedien unter Bedingungen gehalten werden, die das Wachstum des Mikroorganismus für 1 bis 8 Stunden, vorzugsweise 4 bis 6 Stunden, ermöglichen.

8. Verfahren nach einem der Ansprüche 1 bis 5, wobei die beimpften Kulturmedien unter Bedingungen gehalten werden, die das Wachstum des Mikroorganismus für 5 bis 12 Generationszeiten des Mikroorganismus ermöglichen.

9. Verfahren nach einem der vorhergehenden Ansprüche, wobei die mindestens zwei Teilchen elektrisch geladene, magnetische oder magnetisierbare Teilchen oder mit mindestens einer magnetischen oder magnetisierbaren Schicht bedeckte Teilchen sind.

10. Verfahren nach einem der vorhergehenden Ansprüche, wobei die mindestens zwei Teilchen einem elektromagnetischen Feld ausgesetzt werden, das gegebenenfalls in Pulsen angelegt wird.

**11.** Verfahren nach einem der vorhergehenden Ansprüche, bei dem die mindestens zwei Teilchen einem allmählichen Anstieg des elektromagnetischen Feldes unterworfen werden.

**12.** Verfahren nach einem der vorhergehenden Ansprüche, bei dem das Magnetfeld durch Mittel zur Erzeugung eines beweglichen Feldes erzeugt wird.

**13.** Verfahren nach einem der vorhergehenden Ansprüche, bei dem die mindestens zwei Partikel mittels einer Lichtquelle beleuchtet werden, um ihre Bewegung zu erfassen.

**14.** Verfahren nach einem der vorhergehenden Ansprüche, wobei die mindestens zwei Teilchen ein Signal erzeugen.

**15.** Verfahren nach dem vorhergehenden Anspruch, wobei die mindestens zwei Teilchen anregbar, fluoreszierend oder phosphoreszierend oder radioaktiv oder chemilumineszierend sind.

**16.** Verfahren nach einem der vorangehenden Ansprüche, wobei eines der Kompartimente den Mikroorganismus nicht enthält und das Kompartiment eine Kontrolle für das Medium darstellt.


**Claims**

**1.** A process for determining the biofilm-producing capacity of a microorganism, comprising the following steps:

a) introducing at least two particles into culture containers comprising a liquid culture medium suited to the growth of said microorganism, said particles resting on a surface submerged in the culture medium,
b) introducing and independently inoculating the culture medium of the containers obtained in step a) with said microorganism at a concentration of from $1\times10^{-1}$ to $1\times10^{-6}$ McFarland (McF), each medium independently comprising a different concentration of microorganisms,
c) maintaining the inoculated culture media in conditions which enable growth of said microorganism,
d) applying a field capable of moving said at least two particles resting on a surface submerged in the culture medium,
e) determining the biofilm-producing capacity of the microorganism by observing and measuring the aggregation of said particles as follows:

- the absence of aggregation of said particles appearing at a concentration of from $1\times10^{-6}$ to $1\times10^{-4}$ McF corresponding to a strongly biofilm-producing microorganism,
- the absence of aggregation of said particles appearing from a concentration of greater than $1\times10^{-4}$ and less than $1\times10^{-2}$ McF corresponding to a moderately biofilm-producing microorganism,
- the absence of aggregation of said particles appearing from a of greater than or equal to $1\times10^{-2}$ McF corresponding to a weakly biofilm-producing microorganism,
- an aggregation of said particles, regardless of the concentration, corresponding to a non-biofilm-producing microorganism.

**2.** The process according to claim 1, wherein the process comprises a step e') for determining the Biofilm Index (BFI) value for each of the media by:

- analyzing the red, green and blue color components of the pixels of the image, making it possible to determine the particle density per pixel,
- calculating the correlation between the particles,
- calculating the aggregation density of the particles expressed as BFI, and

determining the biofilm formation potential index per container (BPc) according to the following formula (I):

$$BPc = [1-(BFIe / BFIn)] \ (I)$$

wherein BFIe corresponds to the biofilm formation index value in the inoculated medium and BFIn corresponds to the biofilm formation index value in a container which does not comprise microorganisms (negative control).

3. The process according to claim 2, comprising a step e") subsequent to or simultaneous with the step e') for determining the threshold value (S) according to the following formula (II):

$$S= 1 – [(mBFIn – 3 \times stmBFIn)/2] / mBFIn \text{ (II)}$$

wherein mBFIn is equal to the mean of the BFIs of the negative controls, stmBFIn is equal to the calculated standard deviation of the mean of the BFIs of the negative controls.

4. The process according to claim 3, wherein the biofilm-producing capacity of the microorganism is determined by comparing the BPc value as a function of the concentration of microorganisms, as follows:

- a BPc value greater than or equal to the threshold value S from a concentration of microorganisms of from $1 \times 10^{-6}$ to $1 \times 10^{-4}$ McF corresponding to a strongly biofilm-producing microorganism,
- a BPc value greater than or equal to the threshold value S from a concentration of microorganisms of greater than $1 \times 10^{-4}$ and less than $1 \times 10^{-2}$ McF corresponding to a moderately biofilm-producing microorganism,
- a BPc value greater than or equal to the threshold value S from a concentration of microorganisms of $1 \times 10^{-2}$ McF corresponding to a weakly biofilm-producing microorganism,
- a BPc value less than the threshold value S, regardless of the concentration, corresponding to a non-biofilm-producing microorganism.

5. A process for classifying microorganisms, comprising the following steps:

a) introducing at least two particles into culture containers comprising a liquid culture medium suited to the growth of said microorganism, said particles resting on a surface submerged in the culture medium,
b) introducing and independently inoculating the culture medium of the containers obtained in step a) with said microorganism at a concentration of from $1 \times 10^{-1}$ to $1 \times 10^{-6}$ McF, each medium independently comprising a different concentration of microorganisms,
c) maintaining the inoculated culture media in conditions which enable growth of said microorganism,
d) applying a field capable of moving said at least two particles resting on a surface submerged in the culture medium, preferentially with the aim of obtaining an aggregation, preferentially in spot form, of said at least two particles,
e) classifying the microorganism by observing the aggregation of said particles as follows:

- category I: absence of aggregation of said particles appearing at a concentration of from $1 \times 10^{-6}$ to $1 \times 10^{-4}$ McF
- category II: absence of aggregation of said particles appearing from a concentration of greater than $1 \times 10^{-4}$ and less than $1 \times 10^{-2}$ McF corresponding to a moderately biofilm-producing microorganism,
- category III: absence of aggregation of said particles appearing from a concentration of greater than or equal to $1 \times 10^{-2}$ McF corresponding to a weakly biofilm-producing microorganism,
- category IV: aggregation of said particles, regardless of the concentration, corresponding to a non-biofilm-producing microorganism.

6. The process according to any one of the preceding claims, comprising six culture containers in which the concentration of microorganisms at step b) is respectively $1 \times 10^{-6}$ McF, $1 \times 10^{-5}$ McF, $1 \times 10^{-4}$ McF, $1 \times 10^{-3}$ McF, $1 \times 10^{-2}$ McF and $1 \times 10^{-1}$ McF.

7. The process according to any one of the preceding claims, wherein the inoculated culture media are maintained in conditions which enable growth of said microorganism for 1 to 8 hours, preferably 4 to 6 hours.

8. The process according to any one of claims 1 to 5, wherein the inoculated culture media are maintained in conditions which enable growth of said microorganism for 5 to 12 generation times of said microorganism.

9. The process according to any one of the preceding claims, wherein said at least two particles are electrically charged, magnetic or magnetizable particles, or particles covered with at least one magnetic or magnetizable layer.

10. The process according to any one of the preceding claims, wherein said at least two particles are subjected to an electromagnetic field, optionally applied in pulses.

**11.** The process according to any one of the preceding claims, wherein said at least two particles are subjected to a gradual increase in the electromagnetic field.

**12.** The process according to any one of the preceding claims, wherein said magnetic field is generated by moving field generating means.

**13.** The process according to any one of the preceding claims, wherein said at least two particles are illuminated by means of a light source, to detect movement thereof.

**14.** The process according to any one of the preceding claims, wherein said at least two particles generate a signal.

**15.** The process according to the preceding claim, wherein said at least two particles are excitable, fluorescent or phosphorescent or radioactive or chemiluminescent.

**16.** The process according to any one of the preceding claims, wherein one of said compartments does not comprise said microorganism, said compartment representing a control for the medium.

Figure 1

Figure 2

Figure 3

Figure 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2916761

- FR 2866707

**Littérature non-brevet citée dans la description**

- **CHAVANT et al.** *Elsevier Amsterdam,* 16 Février 2007, vol. 68 (3), 605-612
- **MARTIN-ESPADA et al.** *Food science and technology,* vol. 56 (1), 58-61
- **JOE J HARRISON et al.** *Nature protocols,* Juin 2010, vol. 5 (7), 1236-1254
- **BENOIT MR ; CONANT CG ; LONESCU-ZANETTI C ; SCHWARTZ M ; MATIN A.** New Device for High-Throughput Viability Screening of Flow Biofilms. *Appl Env ss,* 2010, vol. 76 (13), 4136-4142
- *Biofiles,* (3.8), 21-24
- **BRANGER A.** Microbiochimie et alimentation. 2012
- **CERCA N ; MARTINS S ; CERCA F ; JEFFERSON KK ; PIER GB ; OLIVEIRA R ; AZEREDO J.** Comparative assessment of antibiotic susceptibility of coagulase-negative staphylococci in biofilm versus planktonic culture as assessed by bacterial enumeration or rapid XTT colorimetry. *J Antimicrob Chemother.,* 2005, vol. 56, 331-6
- **CHAVANT P ; GAILLARD-MARTINIE B ; TALON R ; HÉBRAUD M ; BERNARDI T.** A new device for rapid evaluation of biofilm formation potential by bacteria. *J Microbiol Methods.,* 2007, vol. 68 (3), 605-12
- **CHRISTENSEN, G.D. ; SIMPSON, W.A. ; YOUNGER, J.J. ; BADDOUR, L.M. ; BARRETT, F.F. ; MELTON, D.M. ; BEACHEY, E.H.** Adherence of coagulase-negative staphylococci to plastic tissue culture plates: a quantitative model for the adherence of staphylococci to medical devices. *J. Clin. Microbiol.,* 1985, vol. 22, 996-1006
- **COENYE T ; SPILKER T ; REIK R ; VANDAMME P ; LIPUMA JJ.** Use of PCR analyses to define the distribution of Ralstonia species recovered frompatientswith cystic fibrosis. *J Clin Microbiol,* 2005, vol. 43, 3463-3466
- **COSTERTON, J. W. ; P. S. STEWART ; E. P. GREENBERG.** Bacterial biofilms: a common cause of persistent infections. *Science,* 1999, vol. 284, 1318-1322

- **COSTERTON W ; VEEH R ; SHIRTLIFF M ; PASMORE M ; POST C ; EHRLICH G.** The application of biofilm science to the study and control of chronic bacterial infections. *J Clin Invest,* 2003, vol. 112, 1466-1477
- **CROES S ; DEURENBERG RH ; BOUMANS ML ; BEISSER PS ; NEEF C ; STOBBERINGH EE.** Staphylococcus aureus biofilm formation at the physiologic glucose concentration depends on the S. aureus lineage. *BMC Microbiol.,* 2009, vol. 28 (9), 229
- **DAROUICHE RO.** Treatment of infections associated with surgical implants. *N Engl J Med,* 2004, vol. 350, 1422-1429
- **DE BAERE T ; STEYAERT S ; WAUTERS G et al.** Classification of Ralstonia pickettii biovar 3/'thomasii' strains (Pickett 1994) and of new isolates related to nosocomial recurrent meningitis as Ralstonia mannitolytica sp. nov. *Int J Syst Evol Microbiol.,* 2001, vol. 51, 547-58
- **DE GHELDRE Y ; AVESANI V ; BERHIN C ; DELMÉE M ; GLUPCZYNSKI Y.** Evaluation of Oxoid combination discs for detection of extended-spectrum beta-lactamases. *J Antimicrob Chemother,* Octobre 2003, vol. 52 (4), 591-7
- **DISSEMOND J.** When is a wound chronic?. *Hautarzt,* 2006, vol. 57, 55
- **DJORDJEVIC D ; WIEDMANN M ; MCLANDSBOROUGH LA.** Microtiter Plate Assay for Assessment of Listeria monocytogenes Biofilm Formation. *Appl Env Microbiol,* 2002, vol. 68 (6), 2950-2958
- **DONLAN RM.** Biofilms and device-associated infections. *Emerg Infect Dis,* 2001, vol. 7 (2), 277-81
- **ENG RH ; SMITH SM ; CHERUBIN C.** Inoculum effect of new beta-lactam antibiotics on Pseudomonas aeruginosa. *Antimicrob Agents Chemother.,* 1984, vol. 26 (1), 42-7
- **EXTREMINA CL ; COSTA L ; AGUIAR AL ; PEIXE L ; FONSECA AP.** Optimization of processing conditions for the quantification of enterococci biofilms using microtitre-plates. *J Microbiol Meth,* 2011, vol. 84, 167-173

- **FURUYA EY ; LOWY FD.** Antimicrobial strategies for the prevention and treatment of cardiovascular infections. *Curr Opin Pharmacol,* 2003, vol. 3 (5), 464-469
- **GBEJUADE HO ; LOVERING AM ; WEBB JC.** The role of microbial biofilms in prosthetic joint infections - A review. *Acta Orthopaedica,* 2015, vol. 86 (2), 147-158
- **GOTZ F.** Staphylococcus and biofilms. *Mol Microbiol,* 2002, vol. 43, 1367-1378
- **HENGZHUANG W ; WU H ; CIOFU O et al.** In vivo pharmacokinetics/pharmacodynamics of colistin and imipenem in Pseudomonas aeruginosa biofilm infection. *Antimicrob Agents Chemother,* 2012, vol. 56 (5), 2683-2690
- **HØIBY N. ; CIOFU O. ; BJARNSHOLT T.** Pseudomonas aeruginosa biofilms in cystic fibrosis. *Future Microbiology,* 2010, vol. 5, 1663-1674
- **HØIBY N ; BJARNSHOLT T ; GIVSKOV M et al.** Antibiotic resistance of bacterial biofilms. *Int J Antimicrob Agents,* 2010, vol. 35 (4), 322-332
- **HØIBY N ; CIOFU O ; JOHANSEN HK et al.** The clinical impact of bacterial biofilms. *Int J Oral Sci,* 2011, vol. 3 (2), 55-65
- **JACQUELINE C ; CAILLON J.** Impact of Bacterial Biofilm On The Treatment of Prosthetic Joint Infections. *J Antimicrob Chemother,* 2014, vol. 69, i37-i40
- **JOSEPH LA ; WRIGHT AC.** Expression of Vibrio vulnificus Capsular Polysaccharide Inhibits Biofilm Formation. *J Bacteriol,* 2004, vol. 186 (3), 889-893
- **KURTZ SM ; LAU E ; WATSON H ; SCHMIER JK ; PARVIZI J.** Economic Burden of Periprosthetic Joint Infection in the United States. *J Arthroplasty,* 2012, vol. 27 (8), 61-65
- **KWON AS ; PARK GC ; RYU SY ; LIM DH ; LIM DY ; CHOI CH ; PARK Y ; LIM Y.** Higher biofilm formation in multidrug-resistant clinical isolates of Staphylococcus aureus. *Int J Antimicrob Agents,* 2008, vol. 32, 68-72
- **LATIMER J ; FORBES S ; MCBAIN AJ.** Attenuated virulence and biofilm formation in Staphylococcus aureus following sublethal exposure to triclosan. *Antimicrob Agents Chemother.,* Juin 2012, vol. 56 (6), 3092-100
- **LEID JG ; WILLSON CJ ; SHIRTLIFF ME ; HASSETT DJ ; PARSEK MR ; JEFFERS AK.** The exopolysaccharide alginate protects Pseudomonas aeruginosa biofilm bacteria from IFN-gamma-mediated macrophage killing. *J Immunol.,* 01 Décembre 2005, vol. 175 (11), 7512-8
- **LINDSAY, D. ; VON HOLY, A.** Bacterial biofilms within the clinical setting: what healthcare professionals should know. *J Hosp Infect,* 2006, vol. 64 (4), 313-325
- **MARRONI M ; PASTICCI MB ; PANTOSTI A ; COLOZZA MA ; STAGNI G ; TONATO M.** Outbreak of infusion-related septicemia by Ralstonia pickettii in the Oncology Department. *Tumori,* 2003, vol. 89, 575-6
- **MERRITT JH ; KADOURI DE ; O'TOOLE GA.** Growing and Analyzing Static Biofilms. *Curr Protoc Microbiol,* Juillet 2005
- **MOREMI N ; MUSHI MF ; FIDELIS M ; CHALYA P ; MIRAMBO M ; MSHANA SE.** Predominance of multi-resistant Gram-negative bacteria colonizing chronic lower limb ulcers (CLLUs) at Bugando Medical Center. *BMC Res Notes,* 04 Avril 2014, vol. 7, 211
- **MÜSKEN M ; DI FIORE S ; RÖMLING U ; HÄUSSLER S.** A 96-well-plate-based optical method for the quantitative and qualitative evaluation of Pseudomonas aeruginosa biofilm formation and its application to susceptibility testing. *Nature Protocols,* 2010, vol. 5 (8), 1460-1469
- **NAPARSTEK L ; CARMELI Y ; NAVON-VENEZIA S ; BANIN E.** Biofilm formation and susceptibility to gentamicin and colistin of extremely drug-resistant KPC-producing Klebsiella pneumoniae. *J Antimicrob Chemother,* 2014, vol. 69 (4), 1027-34
- NIH Parent Grant Announcement. *Research on Microbial Biofilms,* 02 Septembre 2014
- **O'GARA JP ; HUMPHREYS H.** Staphylococcus epidermidis biofilms: importance and implications. *J Med Microbiol,* 2001, vol. 50, 582-587
- **OLIVARES E ; BADEL-BERCHOUX S ; PROVOT C ; JAULHAC B ; PRÉVOST G ; BERNARDI T ; JEHL F.** The Biofilm Ring Test®: a rapid method for the routine analysis of P. aeruginosa biofilm formation kinetics. JCM Accepted Manuscript Posted Online. *J. Clin. Microbiol.,* 30 Décembre 2015
- **OTTO M.** Staphylococcal biofilms. *Curr Top Microbiol Immunol,* 2008, vol. 322, 207-228
- **OTTO M.** Staphylococcus epidermidis - the 'accidental' pathogen. *Nat Rev Microbiol.,* Août 2009, vol. 7 (8), 555-67
- **PAN Y ; BREIDT F ; GORSKI L.** Synergistic Effects of Sodium Chloride, Glucose, and Temperature on Biofilm Formation by Listeria monocytogenes Serotype 1/2a and 4b Strains. *Appl Env Microbiol,* 2010, vol. 76 (5), 1433-1441
- **PEETERS E ; NELIS HJ ; COENYE T.** Comparison of multiple methods for quantification of microbial biofilms grown in microtiter plates. *J Microbiol Methods,* Février 2008, vol. 72 (2), 157-65
- **PERIASAMY S ; JOO HS ; DUONG AC ; BACH TH ; TAN VY ; CHATTERJEE SS ; CHEUNG GY ; OTTO M.** How Staphylococcus aureus biofilms develop their characteristic structure. *Proc Natl Acad Sci U S A.,* 2012, vol. 24 (4), 1281-6

- **PODSCHUN R ; ULLMANN U.** Klebsiella spp. as nosocomial pathogens: epidemiology, taxonomy, typing methods and pathogenicity factors. *Clin Microbiol Rev,* 1998, vol. 11, 589-603
- **RAHME LG ; STEVENS EJ ; WOLFORT SF ; SHAO J ; TOMPKINS RG ; AUSUBEL FM.** Common virulence factors for bacterial pathogenicity in plants and animals. *Science,* 1995, vol. 268, 1899-902
- **RAO RS ; KARTHIKA RU ; SINGH SP ; SHASHIKALA P ; KANUNGO R ; JAYACHAN-DRAN S ; PRASHANTH K.** Correlation between biofilm production and multiple drug resistance in imipenem resistant clinical isolates of Acinetobacter baumannii. *Indian J Med Microbiol,* 2008, vol. 26, 333-337
- **ROMLING U ; BALSALOBRE C.** Biofilm infections, their resilience to therapy and innovative treatment strategies. *J Intern Med,* 2012, vol. 272 (6), 541-561
- **RYAN MP ; ADLEY CC.** Ralstonia spp.: emerging global opportunistic pathogens. *Eur J Clin Microbiol Infect Dis,* 2014
- **SANCHEZ CJ JR ; MENDE K ; BECKIUS ML ; AKERS KS ; ROMANO DR ; WENKE JC ; MURRAY CK.** Biofilm formation by clinical isolates and the implications in chronic infections. *BMC Infect Dis.,* 2013, vol. 29 (13), 47
- **SANGETHA S ; ZURAINI Z ; SURYANI S ; SASID-HARAN S.** In situ TEM and SEM studies on the antimicrobial activity and prevention of Candida albicans biofilm by Cassia spectabilis extract. *Micron,* 2009, vol. 40, 439-443
- **SCHABER JA ; CARTY NL ; MCDONALD NA ; GRAHAM ED ; CHELUVAPPA R ; GRISWOLD JA ; HAMOOD AN.** Analysis of quorum sensing-deficient clinical isolates of Pseudomonas aeruginosa. *J Med Microbiol,* 2004, vol. 53, 841-53
- **SINGH, P.K. ; SCHAEFER, A.L. ; PARSEK, M.R. ; MONINGER, T.O. ; WELSH, M.J. ; GREENBERG, E.P.** Quorum-sensing signals indicate that cystic fibrosis lungs are infected with bacterial biofilms. *Nature,* 2000, vol. 407, 762-764
- **SKOGMAN ME ; VUORELA PM ; FALLARERO A.** Combining biofilm matrix measurements with biomass and viability assays in susceptibility assessments of antimicrobials against Staphylococcus aureus biofilms. *J Antibiot,* 2012, vol. 65, 453-459
- **STAPLETON FDJ ; MATHESON M ; WOODWARD E.** Bacterial adherence and glycocalyx formation on unworn hydrogel lenses. *J Brit Contact Lens Assoc,* 1993, vol. 16, 113-6
- **STAPPER AP ; NARASIMHAN G ; OHMAN DE ; BARAKAT J ; HENTZER M ; MOLIN S ; KHARAZMI A ; HØIBY N ; MATHEE K.** Alginate production affects Pseudomonas aeruginosa biofilm development and architecture, but is not essential for biofilm formation. *J Med Microbiol,* Juillet 2004, vol. 53, 679-90
- **STEPANOVIC S ; VUKOVIC D ; DAKIC I ; SAVIC B ; SVABIC-VLAHOVIC M.** A modified microtiter-plate test for quantification of staphylococcal biofilm formation. *J Microbiol Methods,* 2000, vol. 40, 175-9
- **SUETENS C ; MORALES I ; SAVEY A et al.** European surveillance of ICU-acquired infections (HELICS-ICU): methods and main results. *Journal of Hospital Infection,* 2007, vol. 65 (2), 171-173
- **VIERA AJ ; GARRETT JM.** Understanding interobserver agreement: the kappa statistic. *Fam Med,* Mai 2005, vol. 37 (5), 360-3
- **WANG Q ; SUN FJ ; LIU Y ; XIONG LR ; XIE LL ; XIA PY.** Enhancement of biofilm formation by subinhibitory concentrations of macrolides in icaADBCpositive and -negative clinical isolates of Staphylococcus epidermidis. *Antimicrob Agents Chemother,* 2010, vol. 54, 2707-2711
- **WELCH K ; CAI Y ; STRØMME M.** A Method for Quantitative Determination of Biofilm Viability. *J. Funct. Biomater.,* 2012, vol. 3, 418-431
- **YANG D ; ZHANG Z.** Biofilm-forming Klebsiella pneumoniae strains have greater likelihood of producing extended-spectrum beta-lactamases. *J. Hosp. Infect.,* 2008, vol. 68, 369-371
- **ZHANG YQ ; REN SX ; LI HL ; WANG YX ; FU G ; YANG J ; QIN ZQ ; MIAO YG ; WANG WY ; CHEN RS.** Genome-based analysis of virulence genes in a non-biofilm-forming Staphylococcus epidermidis strain (ATCC 12228). *Mol Microbiol,* Septembre 2003, vol. 49 (6), 1577-93